# EUROPEAN PATENT SPECIFICATION

(11) **EP 2 486 137 B1**
(45) Date of publication and mention of the grant of the patent: **30.05.2018**
(21) Application number: 10822318.1
(22) Date of filing: 04.10.2010
(51) Int. Cl.: C12N 15/861, C12N 15/86, C12N 7/00

(54) **REPLICATION-COMPETENT AD11P BASED VIRAL VECTORS**
AUF AD11P BERUHENDE REPLIKATIONS-KOMPETENTE VIRALE VEKTOREN
VECTEURS VIRAUX DÉRIVÉS DU SÉROTYPE AD11P COMPÉTENTS POUR LA RÉPLICATION

(30) Priority: 05.10.2009 US 248516 P
(43) Date of publication of application: 15.08.2012
(73) Proprietor: Mei, Ya-Fang, 907 51 Umeå (SE); Wadell, Göran, 903 39 Umeå (SE)
(72) Inventor: Mei, Ya-Fang, 907 51 Umeå (SE); Wadell, Göran, 903 39 Umeå (SE)
(74) Representative: Novitas Patent AB
(86) International application number: PCT/SE2010/051066
(87) International publication number: WO 2011/043719

(56) References cited:
- EP-A1- 1 785 488
- EP-A1- 1 785 488
- D. STONE ET AL: "Development and Assessment of Human Adenovirus Type 11 as a Gene Transfer Vector", JOURNAL OF VIROLOGY, vol. 79, no. 8, 15 April 2005 (2005-04-15) , pages 5090-5104, XP055052797, ISSN: 0022-538X, DOI: 10.1128/JVI.79.8.5090-5104.2005
- SANDBERG L. ET AL: 'Replication-Competent Adllp Vector (RCAdllp) Efficiently Transduces and Replicates in Hormone-Refractory Metastatic Prostate Cancer Cells' HUMAN GENE THERAPY vol. 20, April 2009, pages 361 - 373, XP008155596
- SILVER J. ET AL: 'Transduction and Oncolytic-Efficacy Profile of RCAd11pGFP, a Potent and Selective Oncolytic Adenovirus 11p Vector' MOLECULAR THERAPY vol. 17, no. 1, May 2009, pages S185 - S186, XP008155595
- MEI Y.-F. ET AL: 'Replication Competent Ad11 Vectors Possess a High Potential for Human Cells Targeted Cancer Therapy' MOLECULAR THERAPY vol. 13, no. 1, 2006, page S54, XP008155598
- HOLTERMAN L. ET AL: 'Novel Replication-Incompetent Vector Derived from Adenovirus Type 11 (Adll) for Vaccination and Gene Therapy: Low Seroprevalence and Non-Cross-Reactivity with Ad5' JOURNAL OF VIROLOGY vol. 78, no. 23, 2004, pages 13207 - 13215, XP008155615 & DATABASE GENBANK [Online] 12 November 2004 EMBL Database accession no. AY598970
- GAO W. ET AL: 'Human adenovirus type 35: nucleotide sequence and vector development' GENE THERAPY vol. 10, 2003, pages 1941 - 1949, XP008155597 & DATABASE GENBANK [Online] 12 December 2003 EMBL Database accession no. AY128640
- DATABASE GENESEQ [Online] 08 November 2002 XP003027948 Retrieved from EBI Database accession no. ABQ76121 & WO 02 053759 A1 (WADELL GOERAN ET AL) 11 July 2002
- MEI Y.-F. ET AL: 'Comparative analysis of the genome organization of human adenovirus 11, a member of the human adenovirus species B, and the commonly used human adenovirus 5 vector, a member of species C' JOURNAL OF GENERAL VIROLOGY vol. 84, 2003, pages 2061 - 2071, XP008155633
- ANDERSSON E.K. ET AL: 'Small-Molecule Screening Using a Whole-Cell Viral Replication Reporter Gene Assay Identifies 2-{[2-(Benzoylamino)Benzoyl]Amino}-Benzoic Acid as a Novel Antiadenoviral Compound' ANTIMICROBIAL AGENTS AND CHEMOTHERAPY vol. 54, no. 9, September 2010, pages 3871 - 3877, XP008155684
- LENNART HOLTERMAN ET AL: "Novel Replication-Incompetent Vector Derived from Adenovirus Type 11 (Adll) for Vaccination and Gene Therapy: Low Seroprevalence and Non-Cross-Reactivity with Ad5", JOURNAL OF VIROLOGY, THE AMERICAN SOCIETY FOR MICROBIOLOGY, US, vol. 78, no. 23, 1 January 2004 (2004-01-01), pages 13207-13215, XP008155615, ISSN: 0022-538X, DOI: 10.1128/JVI.78.23.13207-13215.2004
- D. Stone ET AL: "Development and Assessment of Human Adenovirus Type 11 as a Gene Transfer Vector", Journal of Virology, vol. 79, no. 8, 15 April 2005 (2005-04-15) , pages 5090-5104, XP55052797, ISSN: 0022-538X, DOI: 10.1128/JVI.79.8.5090-5104.2005
- BETT A J ET AL: "AN EFFICIENT AND FLEXIBLE SYSTEM FOR CONSTRUCTION OF ADENOVIRUS VECTORS WITH INSERTIONS OR DELETIONS IN EARLY REGIONS 1 AND 3", PROCEEDINGS OF THE NATIONAL ACADEMY OF SCIENCES, NATIONAL ACADEMY OF SCIENCES, US, vol. 91, 1 September 1994 (1994-09-01), pages 8802-8806, XP002004337, ISSN: 0027-8424, DOI: 10.1073/PNAS.91.19.8802
- A. C. SMITH ET AL: "DNA Genome Size Affects the Stability of the Adenovirus Virion", JOURNAL OF VIROLOGY., vol. 83, no. 4, 26 November 2008 (2008-11-26), pages 2025-2028, XP055296629, US ISSN: 0022-538X, DOI: 10.1128/JVI.01644-08
- ZHANG Q ET AL: "Construction and characterization of a replication-competent human adenovirus type 3-based vector as a live-vaccine candidate and a viral delivery vector", VACCINE, ELSEVIER LTD, GB, vol. 27, no. 8, 18 February 2009 (2009-02-18), pages 1145-1153, XP025924622, ISSN: 0264-410X, DOI: 10.1016/J.VACCINE.2008.12.039 [retrieved on 2009-01-13]
- MEI YA-FANG ET AL: "Complete replication-competent adenovirus 11p vectors with E1 or E3 insertions show improved heat stability", VIROLOGY, ELSEVIER, AMSTERDAM, NL, vol. 497, 3 August 2016 (2016-08-03), pages 198-210, XP029723766, ISSN: 0042-6822, DOI: 10.1016/J.VIROL.2016.07.026

## Description

### FIELD OF THE INVENTION

The present disclosure concerns the field of gene therapy and in particular the use of specific adenoviral vector systems for gene therapy, said vector systems offering enhanced efficiency and specificity for gene delivery. More specifically, the present disclosure also provides replicating-competent adenoviral vector systems carrying one or more inserted heterologous gene. The adenoviral vectors system according to the disclosure are characterized by high binding efficiency and infectivity to cells of neural origin, endothelial cells, carcinoma cells and dendritic cells.

### BACKGROUND OF THE INVENTION

Gene transfer into neural cells has grown into a big field in neuroscience. The usage of gene transfer is ranging from treatment of genetic diseases, tumours and acquired degenerative encephalopaties such as Alzheimer's disease and Parkinson's disease to being a powerful tool in the study of biological mechanisms. An important application of gene transfer is gene therapy, which is when a therapeutic gene is inserted into the cells by ex vivo or in vivo techniques. One of the obstacles to overcome with gene therapy is to get the gene into the right cell type. The choice of cell depends on the nature of the disease. In diseases such as Haemophilia B where a blood clotting factor (IX) is missing in plasma, it is not as important to reach the damaged cell type. Even if the liver normally makes the clotting factor, it does not matter if the therapeutic gene is inserted in muscle cells, fibroblasts or even blood cells as long as the clotting protein is produced in therapeutic amounts and with the correct post-translational modifications. The protein accessibility to its target and the immunological status is also important. A protein that is normally expressed only inside the blood-brain barrier could for example be immunogenic if exposed on the outside. Other emerging areas where gene transfer can become an important therapeutic tool are regenerative and reparative medicine.

There are two different approaches to deliver the DNA (target genes) into the cells. The first is the usage of non-viral vectors to insert the DNA. The non-viral approach consists of methods like direct injection of the DNA, mixing the DNA with polylysine or cationic lipids that allow the DNA to be internalised. Most of these approaches have a low efficiency of delivery and transient expression of the gene. The second and more widely used approach to insert the DNA is by using viral vectors. Viruses have evolved a mechanism to insert their DNA into cells very effectively, but the side effect is that humans have evolved an effective immune response to eliminate viruses from the body.

To function as a viral vector in the nervous system, the vector should have certain properties. Since almost all cells in the brain are non-dividing, the vector must be able to infect non-dividing cells. A good vector also needs to be non-toxic to the cells in the dose required for infection (direct cytotoxicity i.e. by capsid proteins and antigenicity). After the DNA has entered the nucleus it should integrate in a site-specific location in the host chromosome or become a stable extra-chromosomal element (episome). The desired gene should be expressed without interfering with the cellular expression machinery.

Viral vectors used for gene delivery into the nervous system are herpes simplex-1 virus, adenovirus, adeno-associated virus, complex retrovirus, and simple retrovirus such as lentivirus. For this purpose the viruses pathogenicity genes have been deleted and their ability to replicate has-been incapacitated.

Adenoviruses are good candidates for gene therapy towards the nervous system for a number of reasons. They can infect both dividing and non-dividing cells, the viral genome is relatively stable and is easy to manipulate by recombinant DNA techniques. Replication of the virus is efficient in permissive cells and the pathogenicity is low. One of the obstacles to overcome with all viral vectors is to achieve a sustained expression. The viral vector evokes an innate immune response and an adaptive immune response that is both cell mediated and humoral and the infected cells may become destroyed by the inflammatory response within a couple of weeks. The immune response evoked by adenoviral gene transfer is however different in the brain compared to in the peripheral tissues. The immune response in the brain is not sufficiently strong to eradicate the adenovirus infected cells. However, if the individual has had a previous exposure to the adenovirus or was inoculated with the virus later, a strong inflammatory response can be evoked also in the brain. Consequently, adenovirus serotypes of low prevalence in the society should preferentially be used as vectors to be more successful as gene delivery vectors.

The adenoviruses are a family of DNA viruses that can infect both dividing and non-dividing cells. They do not usually integrate into the host chromosome, instead they are replicated as extra-chromosomal elements inside the nucleus of the host cell. Adenoviruses can bind to a range of different cell types. The clinical picture of an adenovirus infection is often respiratory infection or gastro-enteritis. A tonsillitis similar to a streptococcus A infection is also not uncommon. Some of the adenovirus serotypes can cause epidemic keratoconjunctivitis or in some cases even meningitis or encephalitis.

There are at the present 51 known serotypes of adenovirus, which have been divided into six different subgenera, A-F, depending on their biological properties and genetic homology. Virus within the same subgenus shares more than 50% DNA homology whereas viruses in different subgenera have less than 20% homology (Wadell G., Adenoviruses (adenoviridae): General features. Encyclopaedia of Virology. Ed. Webster R. G., Granoff A., Academic Press Ltd London, pp 1-7, 1999).

Human adenoviruses are non-enveloped and about 80 nm in diameter with a 36 Kbp double stranded DNA. The virion capsid is composed of 240 hexon capsomers and 12 vertex capsomers. An antenna-like fibre projects from each vertex capsomer (located at the corners of the icosahedral capsid). The epitopes capable of making serotype specific antibodies and hence also the epitopes determining the serotype are located on the external portions of the hexons and on the most distal knob of the fibre.

Infection starts with the attachment of the fibre knob to a cellular receptor on the permissive cell. The cellular receptor for the virus fibre is coxsackievirus-adenovirus receptor (CAR) for all subgenera except subgenus B. Additional cellular receptors for adenoviruses are CD46, CD80, CD86, sBARand sialic acid. The viral penton base then binds to the cellular integrin α*_{ν}*β₃ and the virus is internalised by endocytosis into an endosome. Upon fusion with a lysosome, the pH is lowered leading to alterations in the viral capsid, releasing the virion from the endo-lysosome. The virion is then transported to the nucleus where the replication and transcription takes place. The spliced mRNA is translated in the cytoplasm. Production of the fibre protein can be detected 9-11 h after infection. The structural proteins are then translocated into the nucleus where assembly of new virions takes place.

Efficient adenovirus infection of target cells as described above depends upon the presence of the coxsackie-adenovirus cell surface receptor, CAR, which is the primary receptor for all human adenoviruses except species B viruses. Several human carcinoma cells lacking CAR have been reported. A lower level of CAR expression has been demonstrated in human bladder cancer cells (Li et al. 1999. Loss of adenoviral receptor expression in human bladder cancer cells: a potential impact on the efficacy of gene therapy. Cancer Res 59(2), 325-30). Prostate cancer specimens with a low-grade Gleason score (GS) and with a high-grade GS or malignant cells showed a similar membrane staining pattern to that of normal tissue, but CAR-specific staining was markedly diminished in high-grade GS specimens (Rauen et al. 2002. Expression of the coxsackie adenovirus receptor in normal prostate and in primary and metastatic prostate carcinoma: potential relevance to gene therapy. Cancer Res 62(13), 3812-8). Thus, the amount of CAR expression in prostate cancer indicated the patient's prognosis. In a mouse model, transfection with CAR increased the efficacy of Ad5 vectors (Bao et al. 2005. Human coxsackie adenovirus receptor (CAR) expression in transgenic mouse prostate tumors enhances adenoviral delivery of genes. Prostate 64(4), 401-7).

The use of adenovirus vectors to control metastasis of cancer has been hampered by the low expression of the CAR receptor. Furthermore, the high sero-prevalence of this adenovirus type in the human population induces adverse reactions in patients. To increase transduction efficacy, Ad5 vectors modified to carry the fiber of a species B adenovirus, or the integrin binding site of RGD or the laminin-derived peptid SIKVAV in their fiber knobs have been successfully used in several cases (Rajecki et al. 2007. Treatment of prostate cancer with Ad5/3Delta24hCG allows non-invasive detection of the magnitude and persistence of virus replication in vivo. Mol Cancer Ther 6(2), 742-51; Stevenson et al. 2007, Incorporation of a laminin-derived peptide (SIKV AV) on polymer-modified adenovirus permits tumor-specific targeting via alpha6-integrins. Cancer Gene Ther 14(4), 335-45). However, the preexisting immunity against Ad5 diminishes the efficacy of the vector and an alternative adenovirus vector that uses a receptor other than CAR represents an attractive option.

Ad11, belonging to species B adenoviruses, uses CD46 and sBAR as the primary receptors (Segerman et al. 2003, There are two different species B adenovirus receptors: sBAR, common to species B1 and B2 adenoviruses, and sB2AR, exclusively used by species B2 adenoviruses. J Virol 77(2), 1157-62; Tuve et al. 2006, A new group B adenovirus receptor is expressed at high levels on human stem and tumor cells. J Virol 80(24), 12109-20). CD46 is upregulated on the surface of tumour cells. An additional benefit of using Ad11 as a vector is the low seroprevalence in humans. Replication-incompetent species B vectors have been reported (Lemckert et al. 2005. Immunogenicity of heterologous prime-boost regimens involving recombinant adenovirus serotype 11 (Ad11) and Ad35 vaccine vectors in the presence of anti-ad5 immunity. J Virol 79(15), 9694-701; Stone et al. 2005. Development and assessment of human adenovirus type 11 as a gene transfer vector. J Virol 79(8), 5090-104) and some limitations of using such vectors have been observed: e.g. the virus can only replicate in its packaging cells and it cannot spread to neighboring tumor cells.

It has previously been shown that adenovirus 11p exhibits high affinity to hematopoietic cells (Segerman et al. 2000. Adenovirus types 11 p and 35 p show high binding efficiencies for committed hematopoietic cell lines and are infective to these cell lines, J Virol 74(3), 1457-1467). This article however gives insufficient information on the interaction between the virus and normal cells or stem cells. Importantly, the experimental set-up results in the information being specific for cancerous cells, and the relevance on non-cancerous or pre-cancerous cells can be disputed.

US patent 7,459,153 and the corresponding EP 1 348 030 disclose the complete nucleic acid sequence of native adenovirus type 11. The sequence is also available through the EBI/EMBL database under accession No. AF532578.

Oncolytic effects of a recombinant adenovirus vector have been described (Sandberg et al. 2009. Replication-competent Ad11p vector (RCAd11p) efficiently transduces and replicates in hormone-refractory metastatic prostate cancer cells. Hum Gene Therapy 20, 361-373). However, this publication does not provide any information as to how this vector was constructed.

Mei Y and Wadell G. 2006. Replication Competent Ad11 Vectors Possess a High Potential for Human Cells Targeted Cancer Therapy. Molecular Therapy 13 Suppl. 1, S54. Reports on replication competent Ad 11E1 and Ad11E3 vectors that can efficiently transduce human cancer cells.

Holterman L et al. 2004. Novel replication-incompetent vector derived from adenovirus type 11 (Ad11) for vaccination and gene therapy: low seroprevalence and non-cross-reactivity with Ad5. J Virol 78,13207-13215, describes an E1 deleted Ad11 vector that on PERC6/55k cells can complement for the deleted adenovirus E1 gene. This incomplete vector could thus be produced in high titers.

Stone et al. 2005. Development and Assessment of Human Adenovirus Type 11 as a Gene Transfer Vector. J Virol 79; 5090-5104, describes an Ad11 vector deleted in position 389-3348, that was generated in 293 cells that can complement the deleted adenovirus E1 gene.

In view of the above, it remains a problem to make available an adenoviral vector with both high infectivity to important cell types, a low prevalence in society, and which allows the insertion of heterologous genes while maintaining replication competence.

Further problems and their solutions will be evident from the description and examples, as read by a person skilled in the art.

### BRIEF DESCRIPTION OF THE INVENTION

The present inventors have found that it is possible to insert heterologous nucleic acid fragments into the genome of the adenovirus Ad11p thereby providing recombinant adenovirus vectors which unexpectedly maintaining replication competence and the present invention relates to a recombinant replication-competent Ad11p adenovirus vector, that comprises an isolated nucleic acid sequence having at least 90%, preferably at least 95%, more preferably at least 98%, and most preferably at least 99% sequence identity to the sequence set forth in SEQ ID NO: 1, and further comprising one or more inserts of a heterologous nucleic acid fragment(s) that is/are (i) either cloned between nucleotide 247 and nucleotide 568 of SEQ ID NO: 1 of such a vector and which vector expresses the entire viral genome or (ii) wherein said heterologous nucleic acid fragment replaces the sequence between nucleotide 28356 and 29482 of SEQ ID NO: 1 in said vector. Accordingly, in one aspect the present disclosure provides an isolated nucleic acid comprising a sequence having at least 90%, preferably at least 95%, more preferably at least 98%, most preferably at least 99% sequence identity to the sequence set forth in SEQ ID NO: 1, further comprising one or more inserts of a heterologous nucleic acid fragment.

In one aspect said insert is a heterologous gene of interest.

In another aspect said insert encodes a siRNA, shRNA, or a micro RNA.

Preferably said heterologous nucleic acid fragment has a size of between 100 and 5,000 base pairs, such as a between 500 to 2,000 base pairs.

Preferably said heterologous nucleic acid fragment is cloned into the E1 region of the Ad11p genome and/or into the E3 region of the Ad11p genome.

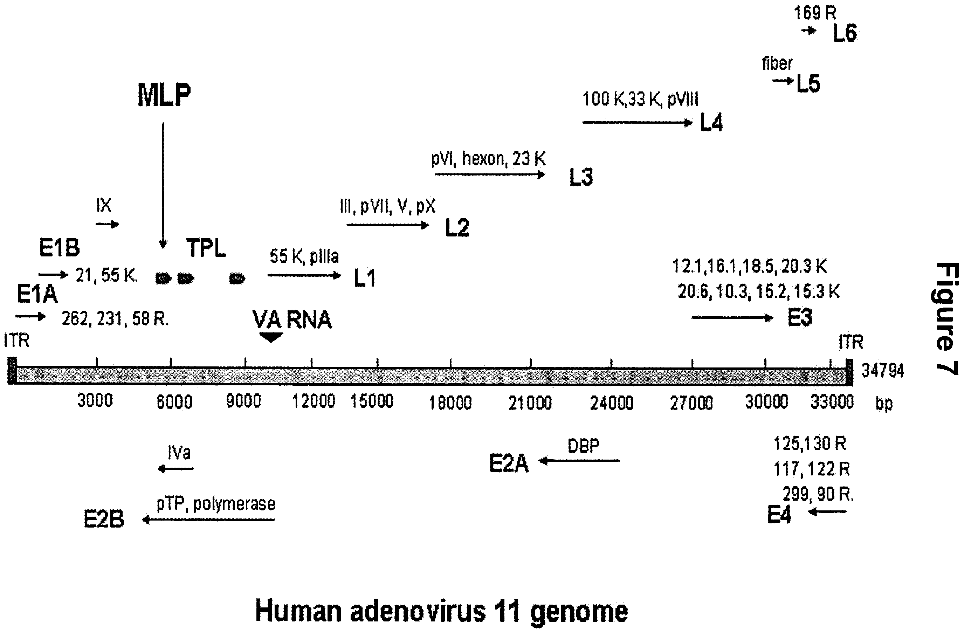

By the E1 region is meant the E1A and E1B regions. The E1A, E1B and E3 regions are defined in Figure 7.

In one preferred aspect one heterologous nucleic acid fragment is cloned into the E1 region, and a second heterologous nucleic acid fragment is cloned into the E3 region.

Preferably said heterologous nucleic acid fragment is cloned into the E1 region at a position corresponding to between nucleotide 247 and nucleotide 568, more preferably between nucleotide 382 and nucleotide 568, even more preferably between nucleotide 382 and nucleotide 479, even more preferably between nucleotide 436 and nucleotide 479 of SEQ ID NO:1.

Most preferably said heterologous nucleic acid fragment is cloned into the E1 region at a position corresponding to nucleotide 247, 382, 436, 479, 451, 479, or 568 of SEQ ID NO:1

Preferably said heterologous nucleic acid fragment is cloned into the E3 region at a position corresponding to between nucleotide 26864 and nucleotide 30633, more preferably between nucleotide 27836 and nucleotide 29526, or even more preferably between nucleotide 27836 and nucleotide 28356, between nucleotide 28356 and nucleotide 29481, between nucleotides 28901 and nucleotide 28919, or between nucleotide 28920 and nucleotide 29526, of SEQ ID NO:1.

Most preferably said heterologous nucleic acid fragment is cloned into the E3 region at a position corresponding to nucleotide 27836 or nucleotide 28356 of SEQ ID NO:1.

In one aspect part of the E3 region is replaced by the insertion of said heterologous nucleic acid fragment.

In one aspect the expression of said heterologous nucleic acid fragment is under the control of a heterologous enhancer and/or promoter.

Preferably said heterologous enhancer and/or promoter is selected from heterologous viral promoters, and mammalian tissue specific promoters.

By mammalian tissue specific promoter is meant a promoter mediating the expression of the heterologous nucleic acid fragment mainly specifically in one or more mammalian tissue.

In another aspect the expression of said heterologous nucleic acid fragment is under the control of adenoviral endogenous gene expression machinery or an adenoviral endogenous enhancer and/or promoter.

In one embodiment said heterologous nucleic acid fragment encodes a protein selected from the group consisting of viral proteins, antigenic determinants of a pathogenic organism, tumour-specific antigens, human therapeutic proteins, and cytokines.

Preferably said heterologous nucleic acid fragment encodes a viral protein selected from the group consisting of ADP, E1A, and p300.

Preferably said heterologous nucleic acid fragment encodes a human protein selected from the group consisting of: Rb, CFTR, pl6, p21, p27, p57, p73, C-CAM, APC, CTS-1, zacl, scFV ras, DCC, NF-1, NF-2, WT-1, MEN-I, MEN-II, BRCA1, VHL, MMAC1, FCC, MCC, BRCA2, IL-1, IL-2, IL-3, IL-4, IL-5, IL-6, IL-7, IL-8, IL-9, IL-10, IL-11 IL-12, GM-CSF, G-CSF, thymidine kinase, mda7, fus, interferon α, interferon β, interferon γ, p53, ABLI, BLC1, BLC6, CBFA1, CBL, CSFIR, ERBA, ERBB, EBRB2, ETS1, ETS2, ETV6, FGR, FOX, FYN, HCR, HRAS, JUN, KRAS, LCK, LYN, MDM2, MLL, MYB, MYC, MYCL1, MYCN, NRAS, PIM1, PML, RET, SRC, TALI, TCL3, YES, MADH4, RBI, TP53, WT1, TNF, BDNF, CNTF, NGF, IGF, GMF, aFGF, bFGF, NT3, NT5, ApoAI, ApoAIV, ApoE, RaplA, cytosine deaminase, Fab, ScFv, BRCA2, zacl, ATM, HIC-1, DPC-4, FHIT, PTEN, ING1, NOEY1, NOEY2, OVCA1, MADR2, 53BP2, IRF-1, Rb, zacl, DBCCR-1, rks-3, COX-1, TFPI, PGS, Dp, E2F, ras, nzyc, neu, raf, erb, fins, trk, ret, gsp, hst, abl, VEGF, FGF, PEDF, thrombospondin, BAI-1, GDAIF, and MCC.

In another embodiment said heterologous nucleic acid fragment encodes a reporter protein selected from the group consisting of: fluorescent proteins, luminescent proteins, and enzymes.

Preferably said reporter protein is selected from the group consisting of green fluorescent protein, enhanced green fluorescent protein, yellow fluorescent protein, blue fluorescent protein, cyan fluorescent protein, red fluorescent protein, luciferase, β-galactosidase, chloramphenicol acetyltransferase.

In another aspect the present invention provides a recombinant replication competent adenovirus vector as defined by the claims comprising a nucleic acid as defined by the claims. Accordingly one important aspect of the present invention is a recombinant replication competent adenovirus vector comprising a sequence having at least 90%, preferably at least 95%, more preferably at least 98%, most preferably at least 99% sequence identity to the sequence set forth in SEQ ID NO:1, further comprising one or more inserts of a heterologous nucleic acid fragment, wherein said heterologous nucleic acid fragment is cloned into the E1 region of the Ad11p genome as defined in the claims and/or into the E3 region of the Ad11p genome. as defined in the claims. By the E1 region is meant the E1A and E1B regions. The E1A, E1B and E3 regions are defined in Figure 7.

Preferably said heterologous nucleic acid fragment is cloned into the E1 region at a position corresponding to between nucleotide 247 and nucleotide 568, more preferably between nucleotide 382 and nucleotide 568, even more preferably between nucleotide 382 and nucleotide 479, even more preferably between nucleotide 436 and nucleotide 479 of SEQ ID NO:1.

Most preferably said heterologous nucleic acid fragment is cloned into the E1 region at a position corresponding to nucleotide 247, 382, 436, 479, 451, 479, or 568 of SEQ ID NO:1

Preferably said heterologous nucleic acid fragment is cloned into the E3 region at a position corresponding to between nucleotide 26864 and nucleotide 30633, more preferably between nucleotide 27836 and nucleotide 29526, or even more preferably between nucleotide 27836 and nucleotide 28356, between nucleotide 28356 and nucleotide 29481, between nucleotides 28901 and nucleotide 28919, or between nucleotide 28920 and nucleotide 29526, of SEQ ID NO:1.

Most preferably said heterologous nucleic acid fragment is cloned into the E3 region at a position corresponding to nucleotide 27836 or nucleotide 28356 of SEQ ID NO:1.

In yet another aspect the present invention provides pharmaceutical compositions comprising a recombinant replication competent adenovirus virus according to the invention, optionally in combination with a suitable excipient.

In yet another aspect the present invention provides recombinant replication competent adenovirus vector according to the invention for use as a medicament.

In yet another aspect the disclosure provides use of a recombinant replication competent adenovirus vector according to the invention in the manufacture of a medicament for the therapeutic, prophylactic or diagnostic treatment of a disease.

Preferably said disease is selected from the group consisting of a viral infectious diseases, bacterial infectious diseases, cancer diseases, vascular diseases, cardiovascular diseases, immunological diseases, liver diseases, and parasitic diseases.

Preferably said viral infectious disease is selected from the group consisting of viral diseases caused by HCV, HBV, HIV, and influenza A virus.

Preferably said bacterial infectious disease is selected from the group consisting of tuberculosis, and septic chock.

Preferably said parasitic disease is malaria.

Preferably said cancer disease is selected from the group consisting of: lung cancer, breast cancer, ovarian cancer, prostate cancer, colorectal cancer, bladder cancer, kidney cancer, pancreatic cancer, haematological malignancies, liver cancer, tumours of neural origin, head and neck cancer, and osteosarcoma.

In yet another aspect the disclosure provides methods of treating a mammalian patient having a cancer disease, said method comprising administering to the patient the pharmaceutical composition according to the disclosure. According to one instance the pharmaceutical composition is administered locally to a tumour site.

According to another instance the pharmaceutical composition is administered intravesically or intraductally.

Intravesical administration can be a therapeutic advantage e.g. in the treatment of bladder cancer. Intraductal administration can be a therapeutic advantage e.g. in the treatment of breast cancer.

According to yet another instance the pharmaceutical composition is administered by intravenous injection.

According to one instance the method further comprises administering a chemotherapeutic agent to the patient.

According to another instance the method further comprises treating the patient with radiation therapy.

In yet another aspect the present disclosure provides use of a recombinant replication competent adenovirus according to the disclosure for gene therapy.

In yet another aspect the present invention provides a mammalian cell comprising a recombinant replication competent adenovirus according to the invention. Transfected mammalian cells can be used for the production of the vectors according to the invention, and for the production of recombinant proteins.

In yet another embodiment the present invention provides methods of expressing a protein or gene product or an expression product which comprises infecting or transfecting a cell in vitro with a recombinant replication competent adenovirus according to the present invention and optionally extracting, purifying or isolating the protein, gene product or expression product from the cell.

In yet another embodiment the present invention provides use of a recombinant replication competent adenovirus vector according to the present invention for the identification of anti-viral compounds.

In yet another embodiment the present invention provides methods for the *in vitro* identification of anti-viral compounds comprising the use of a recombinant replication competent adenovirus vector according to the present invention.

It is contemplated that any method or composition described herein can be implemented with respect to any other method or composition described herein.

The use of the word "a" or "an" when used in conjunction with the term "comprising" in the claims and/or the specification may mean "one," but it is also consistent with the meaning of "one or more," "at least one," and "one or more than one."

These, and other, embodiments of the invention will be better appreciated and understood when considered in conjunction with the following description and the accompanying drawings. It should be understood, that the following description, while indicating various embodiments of the invention and numerous specific details thereof, is given by way of illustration.

### BRIEF DESCRIPTION OF THE DRAWINGS

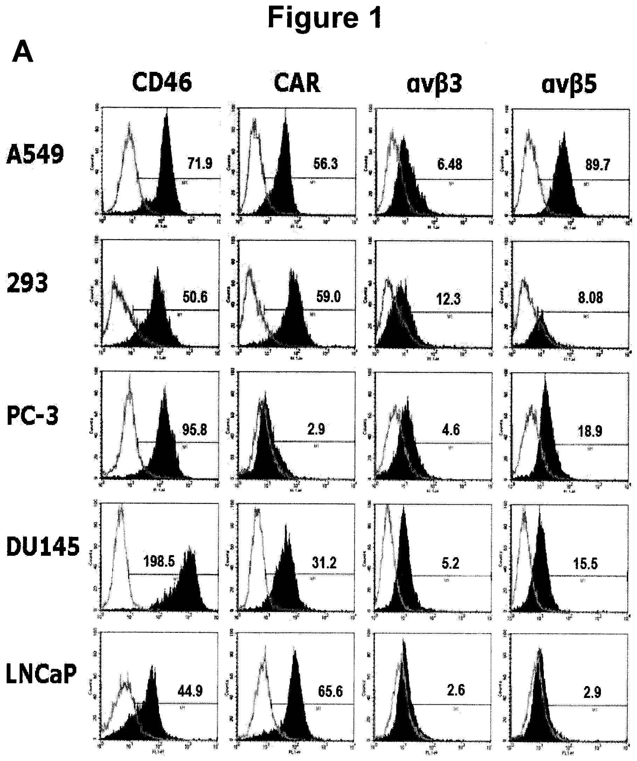

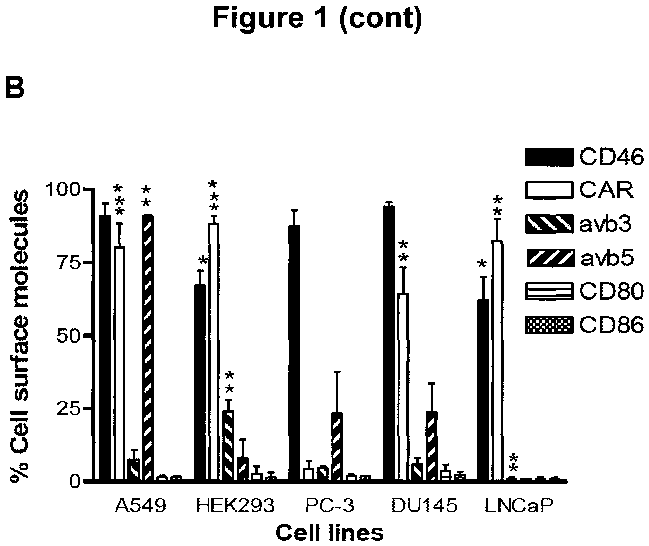

The present invention will be described in further detail below, in the description and attached sequence listing and drawings, in which
**Figure 1****. A.** Flow cytometric analysis of expression of human CD46, CAR, αᵥβ₃ integrin, and αᵥβ₅ integrin on the surface of metastatic cells derived from human prostate (PC-3, DU145, and LNCaP) and control cell lines (A549 and HEK293). **B.** Graph of expression of different cell-surface molecules (CD46, CAR, αᵥβ₃, αᵥβ₅, CD46, CD80, and CD86) in the prostate cancer cell lines PC-3, DU145, and LNCaP, and in the control cell lines A549 and HEK293. Significant difference to PC-3 cells is indicated on top of each bar, *P <0.05; **P<0.01; *** P<0.001.

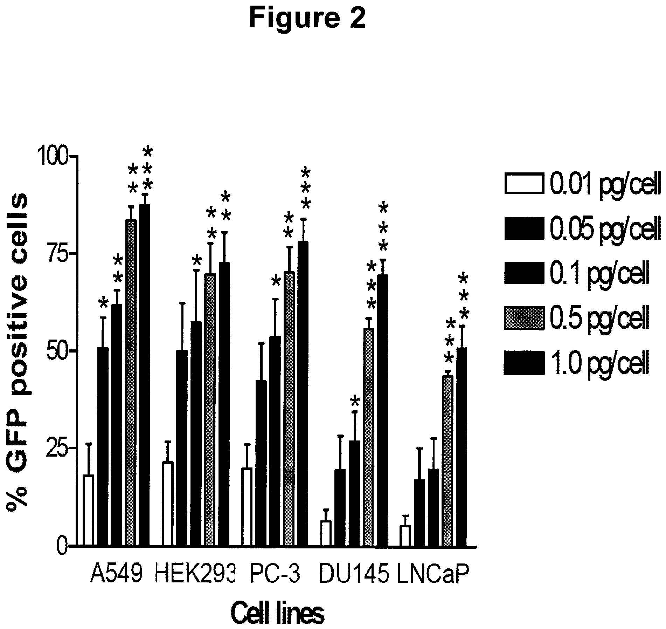

**Figure 2****.** Transduction analysis of prostate carcinoma cell lines (DU145, PC-3, and LNCaP) and control cell lines (A549 and HEK293). The cells were infected with RCAd11pGFP at 0.01, 0.05, 0.1, 0.5, and 1 pg virus particles per cell. The transduction efficiency was assessed by flow cytometry of GFP expression at 48 h postinfection, respectively. Significant difference to 0.01 pg virus per cell is indicated on top of each bar, *P <0.05; **P<0.01; ***P<0.001.

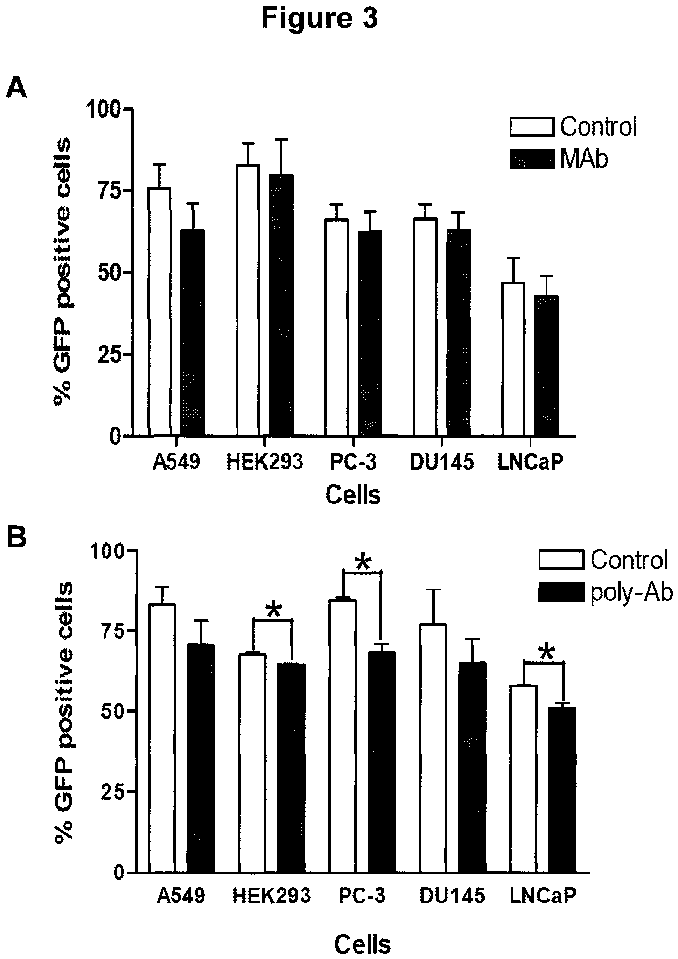

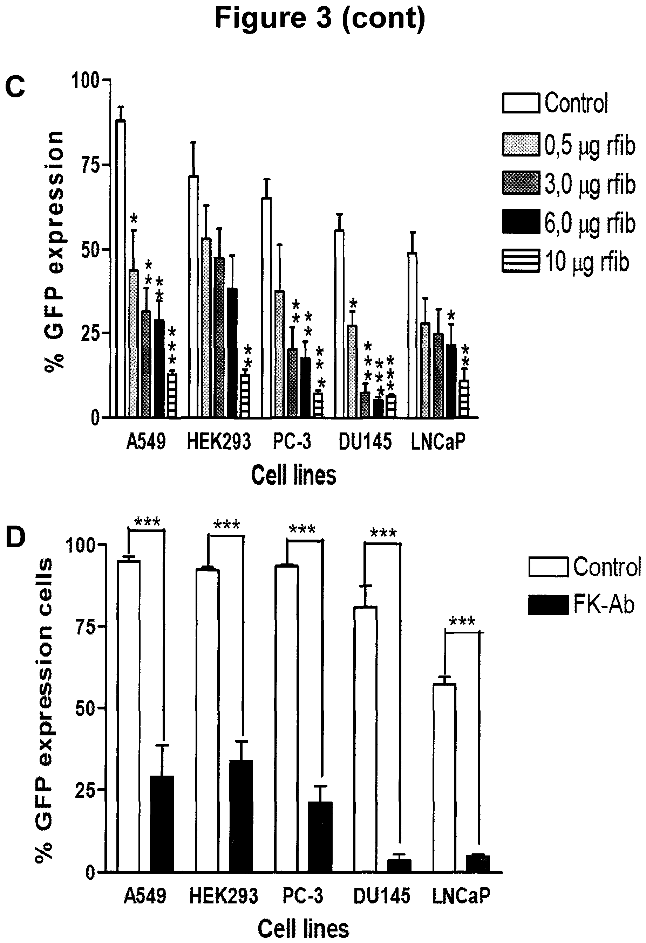

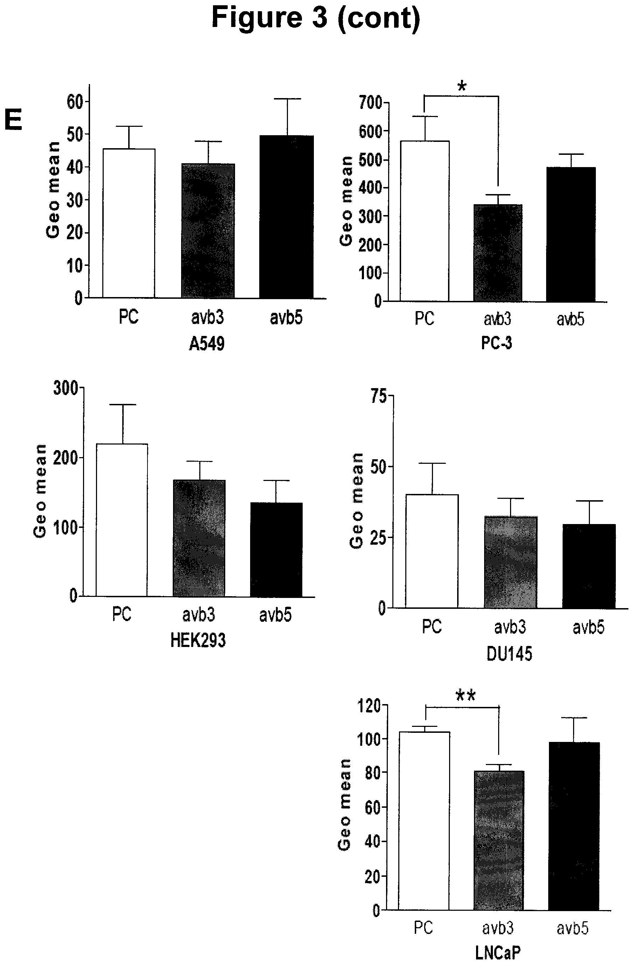

**Figure 3****.** Transduction was reduced by antibodies to CD46, by Ad11prfib, and integrins αᵥβ₃ and αᵥβ₅. **A.** Monoclonal anti-CD46 antibody. **B.** Polyclonal anti-CD46 antibody. **C.** Graph of blockage by Ad11prfib at different concentrations (µg/10⁶ cells). Positive controls were infected with RCAd11pGFP vector at concentrations of 0.5 pg per cell for A549 and 1.0 pg per cell for 293, DU145, PC-3 and LNCaP. **D.** Anti-Adl 1prfib antiserum. **E.** Monoclonal αᵥβ₃ antibody and αᵥβ₅antibody. Significant difference to control is indicated on top of each bar, *P <0.05; **P<0.01; ***P<0.001.

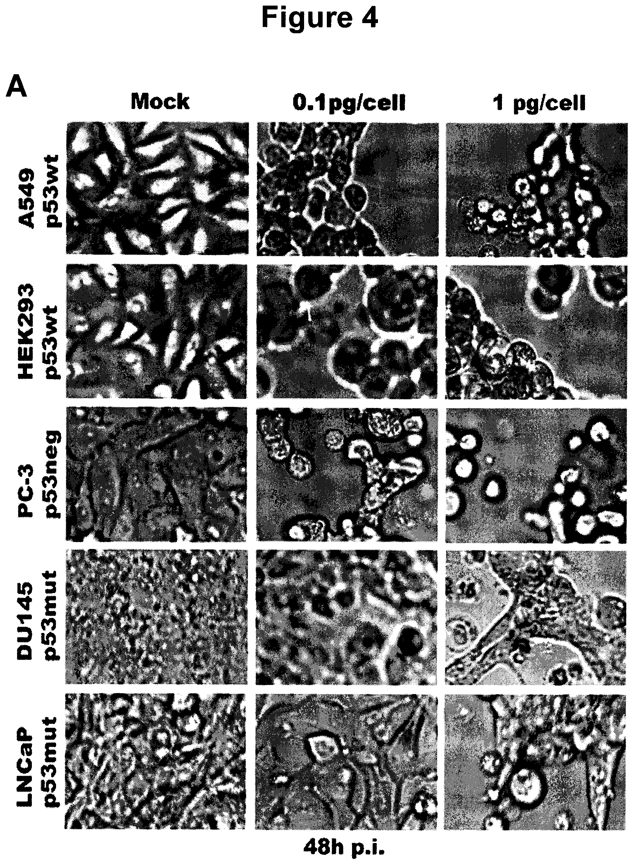

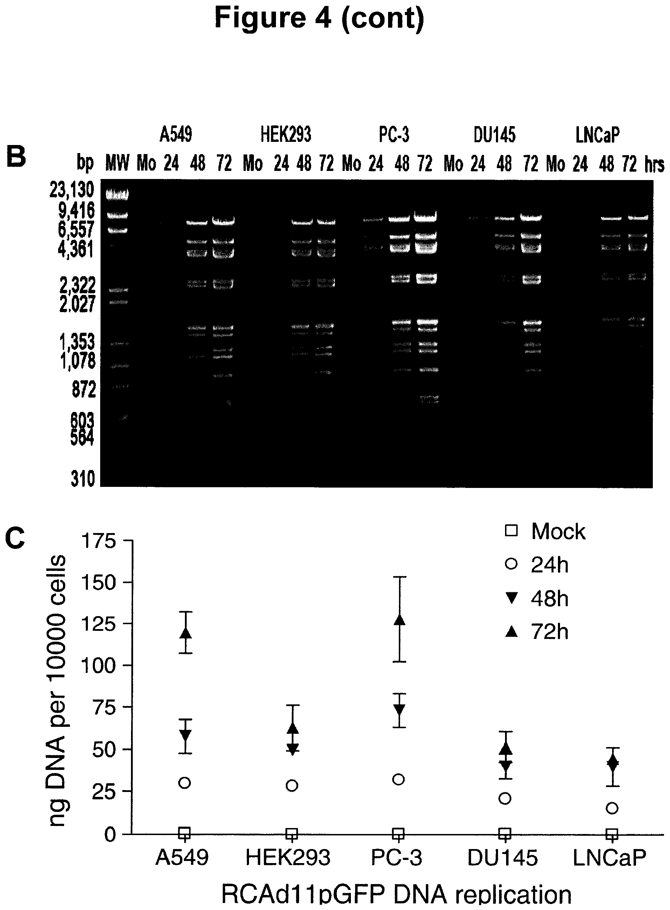

**Figure 4****. A.** The extent of RCAd11p-induced cytopathic effect (CPE) did not correlate with p53 genotype. RCAd11p-induced cytolysis was detected in the p53-positive A549 and HEK293 lines, and in the p53-negative PC-3 line and the p53 mutant DU145 and LNCaP cell lines. Infection was done at 0.1 pg/cell and 1 pg/cell as described in Materials and Methods. Dose-dependent induction cell CPE was evaluated by microscopy at 48 h p.i. **B.** RCAd11p viral DNA replication was assessed in p53⁺ and p53⁻ cell lines. Prostate carcinoma cells (PC-3, DU145, and LNCaP) and control cells (A549 and HEK293) were mock-infected and infected with RCAd11pGFP at 0.1 pg/cell (multiplicity of infection of 5) as described in Materials and Methods. Viral DNA was harvested from infected cells at the indicated times postinfection (p.i.) DNA isolated from an equal number of cells (2 × 10⁵) was digested with the restriction endonuclease BamHI. **C.** The viral DNA was detected using a NanoDrop spectrophotometer.

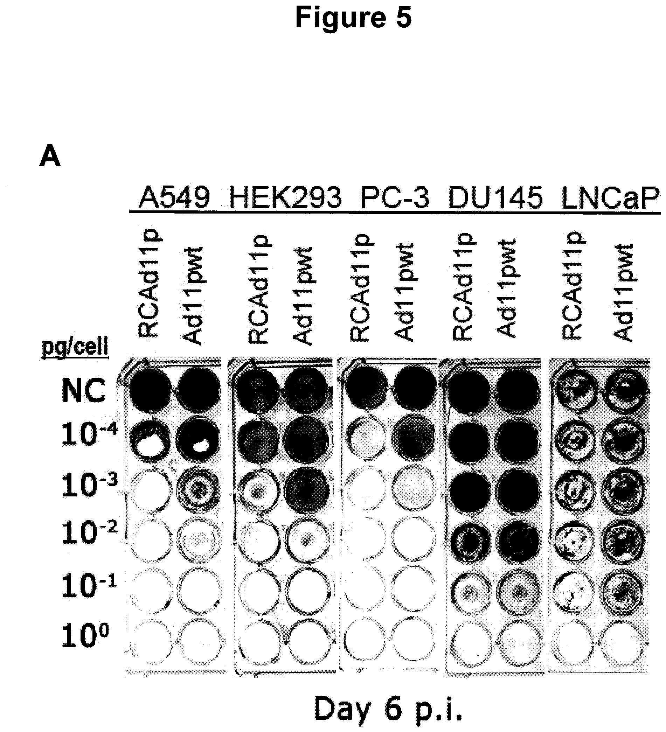

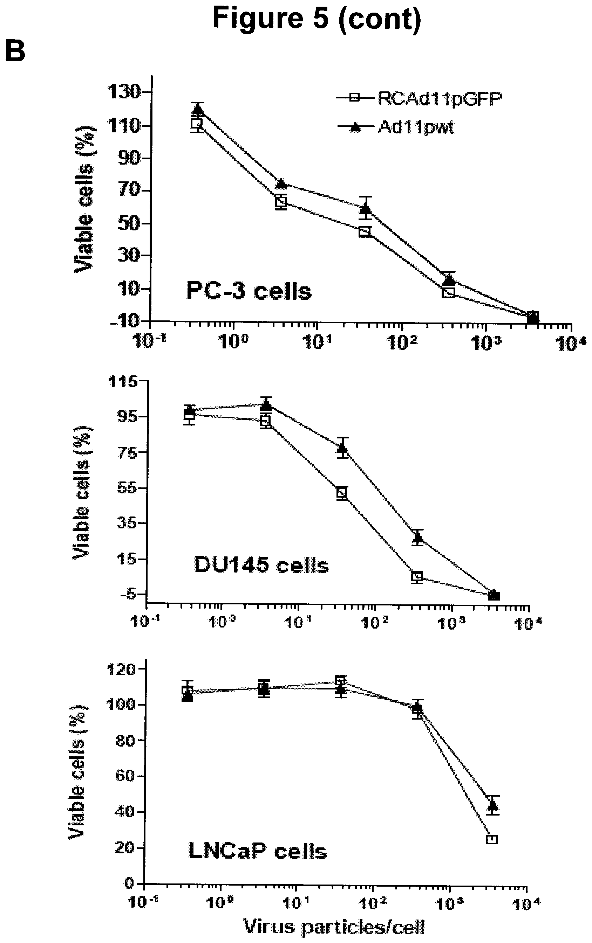

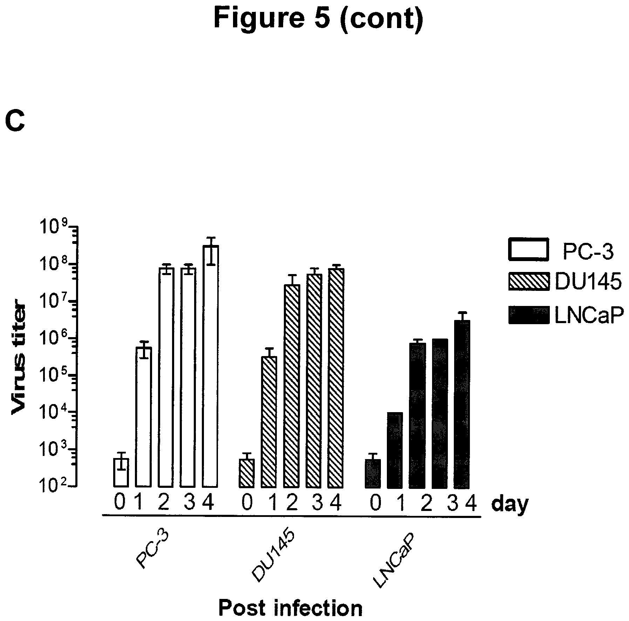

**Figure 5****. A:** Comparison of the oncolytic effect of RCAd11pGFP and Ad11pwt. A549, 293, PC-3, DU145, and LNCaP cells were infected with 10-fold diluted viruses, starting at 1 pg/cell in all tests. The cells were stained by crystal violet 6 days post infection. The RCAd11pGFP virus was slightly more oncolytic than wild-type Ad11. **B.** Cell viability was measured by XTT assay 6 day post infection. The results from XTT assays were presented as absorbance at 490 nm. The graph represents the average of four replicate samples. **C.** One-step grow curve indicated the replication capacity of the virus in the three prostate cancer cell lines.

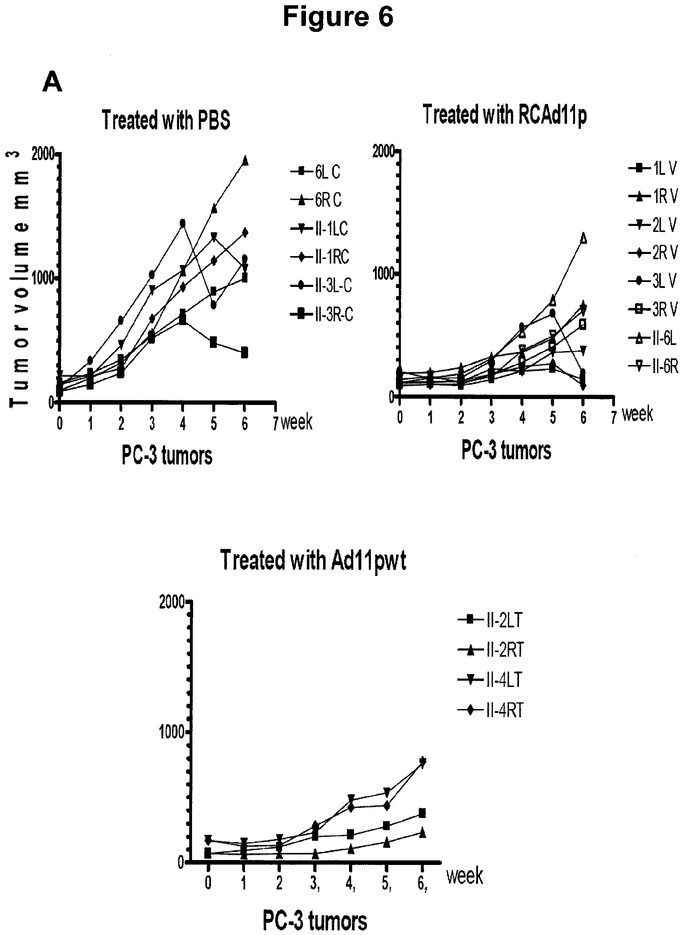

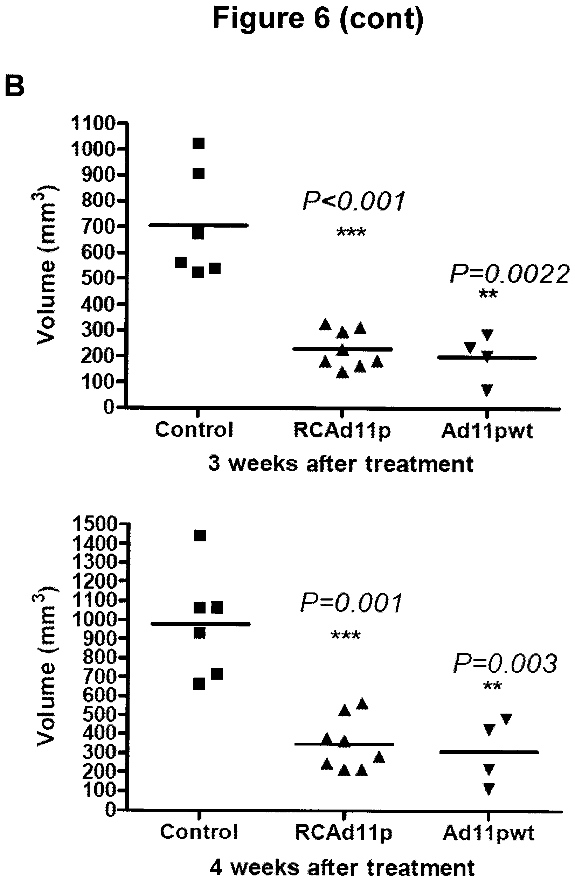

**Figure 6****.** In vivo oncolytic model with RCAd11p vector. 10⁷ PC-3 cells /tumor were subcutaneously transplanted into left and right frank region of *Balb*/*c* nude mice, the control group mice were injected with only PBS. As PC-3 tumor grew at least 75 mm3 after 3 week injection, 50 µg of RCAd11p/tumor was injected into mice with intratumor administration. *A*. tumor volume with or mock treated by RCAd11p was recorded weekly. The mice were scarified approximately 6 weeks after viral injection. ***B*.** statistics of the tumour growths 3 and 4 weeks post infection.
**Figure 7****.** Genome organization of Ad11. The linear double-stranded genome is depicted in the centre as a double line, with the inverted terminal repeats (ITRs) at each end. Transcription units are shown as arrows, relative to their position and orientation in the Ad11 genome. These include early genes (E1A, E1B, E2A, E2B, E3 and E4), genes expressed at intermediate times of infection (IX and IVa2) and late genes (LI-L6). All of the late genes are expressed from the major late promoter (MLP) and contain the tripartite leader (TPL) at their 5'-ends. A triangle represents the virus-associated (VA) RNA. One map unit (m.u.) is equivalent to 347,94 bp.

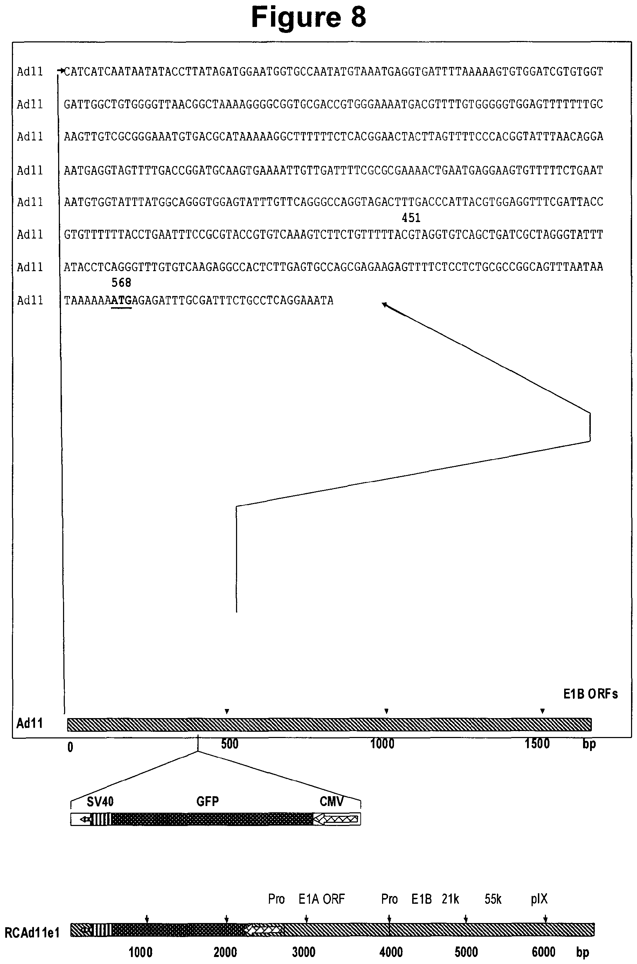

**Figure 8****.** Construction of the RCAd11pGFP vector.
   For the construction of replication competent adenovirus vectors, three alternative insertion sites ain the E1 region of the genome were identified. A foreign gene can be inserted at the beginning of adenovirus genome between nucleotide 1 and nucleotide 568. However, the insertion at the location between nucleotide 247 and nucleotide 568 would produce a competent vector of better quality. Furthermore, insertion of the foreign gene between nucleotides 382 and nucleotide 568 of the adenovirus genome will result in an even better infectious vector. The insertion of the foreign gene between nucleotide 436 and nucleotide 479 or even more preferably at the site corresponding to nucleotide 451 can consequently yield best infectious adenovirus vectors that have a replication competence comparable to their wild type counterparts.

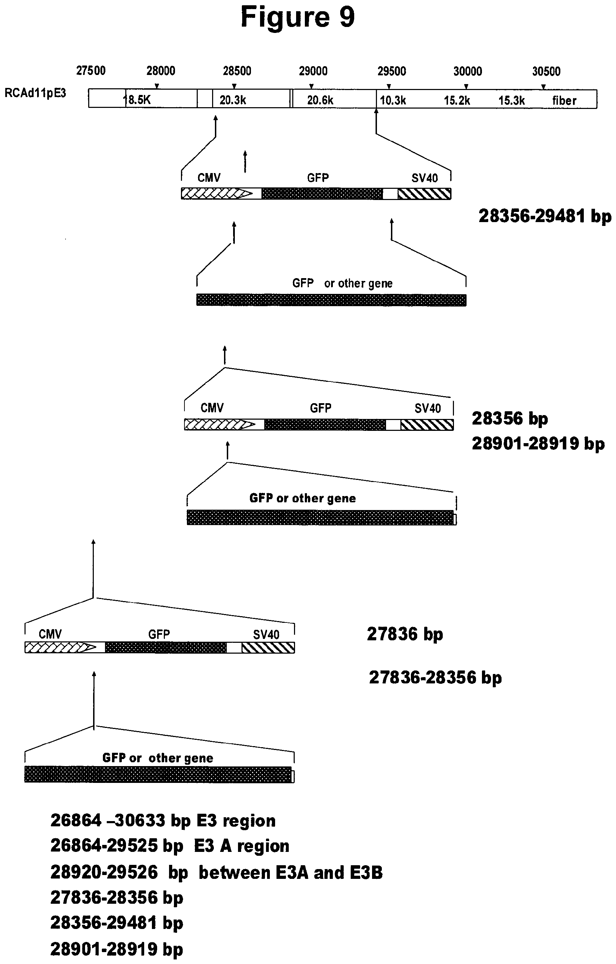

**Figure 9****.** Examples RCAd11p vectors with insertions in the E3 region.
   To generate replication competent adenovirus vector in the E3 region without negative effect on the replication capacity, the entire e3 region between nucleotide 26864 and nucleotide 30633bp or the region between nucleotide 26864 and nucleotide 29525 can be replaced with one open reading frame (ORF) or one expression cassette. Alternatively, insertion of an ORF between nucleotide 28901 and nucleotide 28919 generates infectious adenovirus vectors. Furthermore, the ORF between nucleotide 27836 and nucleotide 28356 or between nucleotide 28356 and nucleotide 29482 can be replaced by one new ORF or one expression cassette so that the new constructs can function as wild type virus. All described constructs consequently produce replication-competent adenovirus vectors without effect on replication capacity.

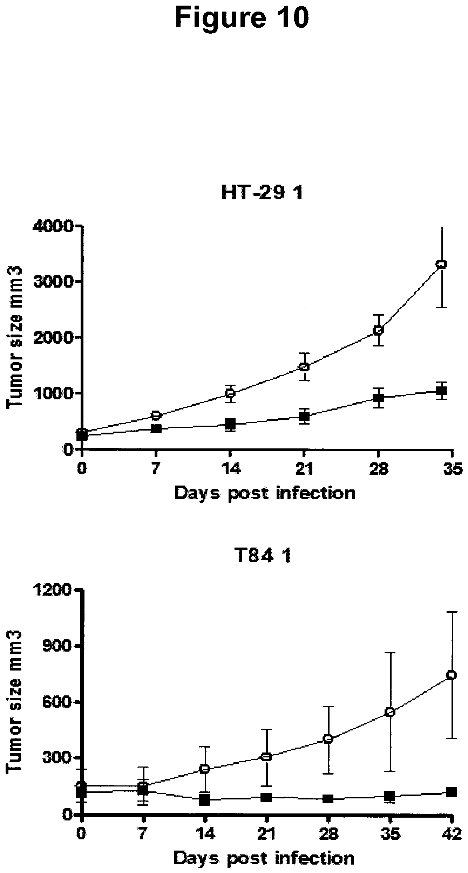

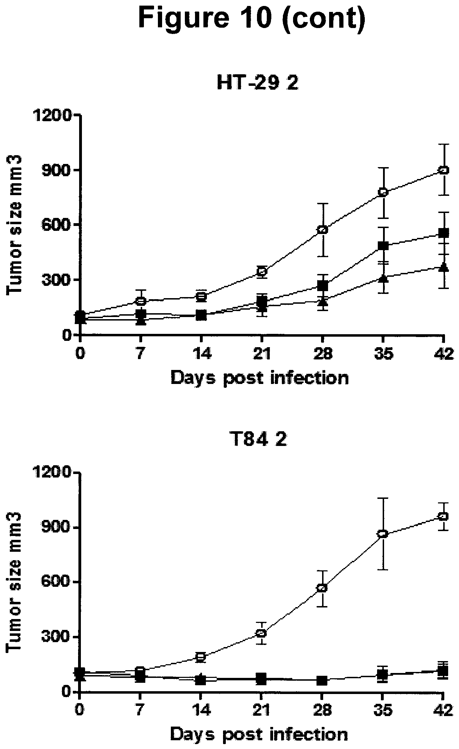

**Figure 10****.** In vivo oncolytic model with RCAd11p vector. T84 cells derived from a lung metastasis of a colorectal adenocarcinoma and HT 29 derived from colorectal adenocarcinoma. 10⁷ T84 cells /tumor or H-29 cells in 0.2 ml were subcutaneously transplanted into left and right flank region of *Balb*/*c* nude mice, the control group mice were injected with only PBS. As colon tumor grew up to at least 75 mm³ 3 weeks after injection, 50 µg of RCAd11p/tumor was injected into mice with intratumoral administration. Tumor volume was recorded weekly. The mice were sacrificed approximately 6 weeks after viral injection, (o) control, (■) mice treated with RCAd11pGFP, (▲) mice treated with wild-type Ad11p.

### DETAILED DESCRIPTION OF THE INVENTION

The term "substantially homologous" as used herein refers to the ability of two nucleic acids to hybridise under at least moderately stringent hybridisation conditions. Stringency of hybridisation is a term of the art that refers to the conditions used for a hybridisation reaction whereby complementary single strands of nucleic acid join to one another to form double-stranded nucleic acid with some degree of mismatch, the degree of which is a function of the stringency-used. In particular, the stringency will depend upon the size and composition of the strands of nucleic acid that are caused to react, the degree of mismatching allowed, the desired cross reactivity, and the like. The degree of stringency can be affected by ionic conditions employed and temperature, among others, as is well known in the art (e.g. Sambrook et al., Molecular cloning: A laboratory manual, second edition, 1989).

The terms "functionally homologous" and "functionally similar" refers to homologies and similarities accounting for the same function or behaviour with respect to tropism, affinity to specific cells and immune response inducing behaviour.

The present inventive adenoviral vector preferably further comprises heterologous nucleic acid fragments, which will typically encode, and express within a host cell, a product that has therapeutic and/or prophylactic utility. The term "heterologous nucleic acid fragment" is used herein to refer to any sequence of DNA or RNA, in particular DNA, functionally inserted into a vector according to the present invention that is foreign to the adenoviral genome. Such heterologous nucleic acid fragment may constitute a gene, a portion of a gene, or any other nucleic acid sequence, including but not limited to a sequence that encodes RNA, anti-sense RNA, a synthetic oligonucleotide, and/or a polypeptide. Heterologous nucleic acid fragment having therapeutic utility include genes that encode a missing or impaired gene function, and genes influencing the behaviour of the cell, such as so called suicidal genes. Foreign nucleic acids having prophylactic utility include genes that encode a gene product that has an ability to prevent disease directly or indirectly, e.g. by providing a source of a polypeptide or other antigen to elicit an immune response thereto.

The term "replication competent" as used herein refers to the ability of a vector to replicate in the host cell.

The term "therapeutic and/or prophylactic agent" and the term "product having therapeutic and/or prophylactic utility" are used as equivalents and are meant to comprise inter alia antigens and immunostimulating agents, such as cytokines etc.

The present disclosure further provides the purified and isolated DNA of an adenovirus having the nucleotide sequence of SEQ ID NO: 1 or a sequence hybridising thereto under stringent conditions or substantially homologous therewith, further comprising an inserted heterologous nucleic acid fragment.

The present invention provides a novel adenoviral as defined by the claims comprising a sequence with at least 90% such as at least 95% identity to the sequence of SEQ ID NO: 1, in particular an adenoviral vector comprising a sequence with at least 98% identity such as at least 99% identity to the sequence of SEQ ID NO: 1 or most preferably an adenoviral vector comprising a sequence substantially homologous to the sequence of SEQ ID NO: 1, further comprising an inserted heterologous nucleic acid fragment.

To determine the percent identity of two nucleic acids, the sequences are aligned for optimal comparison purposes (e.g., gaps can be introduced in the sequence of a first nucleic acid sequence for optimal alignment with a second amino or nucleic acid sequence). The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences (i.e., percent identity = number of identical positions/total number of positions (e.g., overlapping positions) x 100). In one embodiment, the two sequences are the same length.

To determine percent sequence identity, a target nucleic acid sequence is compared to the identified nucleic acid sequence using the BLAST 2 Sequences (B12seq) program from the stand-alone version of BLASTZ containing BLASTN version 2.0.14. This stand-alone version of BLASTZ can be obtained from the U.S. government's National Center for Biotechnology Information web site (world wide web at ncbi.nlm.nih.gov). Instructions explaining how to use the B12seq program can be found in the readme file accompanying BLASTZ.

B12seq performs a comparison between two sequences using either the BLASTN or BLASTP algorithm. BLASTN is used to compare nucleic acid sequences, while BLASTP is used to compare amino acid sequences. To compare two nucleic acid sequences, the options are set as follows: -i is set to a file containing the first nucleic acid sequence to be compared (e.g., C:\seq1.txt); -j is set to a file containing the second nucleic acid sequence to be compared (e.g., C:\seq2.txt); -p is set to blastn; -o is set to any desired file name (e.g., C:\output.txt); -q is set to -1; -r is set to 2; and all other options are left at their default setting. The following command will generate an output file containing a comparison between two sequences: C:\B12seq -i c:\seq1.txt-j c:\seq2.txt -p blastn -o c:\output.txt -q -1 -r 2. If the target sequence shares homology with any portion of the identified sequence, then the designated output file will present those regions of homology as aligned sequences. If the target sequence does not share homology with any portion of the identified sequence, then the designated output file will not present aligned sequences.

Once aligned, a length is determined by counting the number of consecutive nucleotides from the target sequence presented in alignment with sequence from the identified sequence starting with any matched position and ending with any other matched position. A matched position is any position where an identical nucleotide is presented in both the target and identified sequence. Gaps presented in the target sequence are not counted since gaps are not nucleotides. Likewise, gaps presented in the identified sequence are not counted since target sequence nucleotides are counted, not nucleotides from the identified sequence.

The percent identity is determined by dividing the number of matches by the length of the sequence set forth in an identified sequence followed by multiplying the resulting value by 100. For example, if a sequence is compared to the sequence set forth in SEQ ID NO:1 (the length of the sequence set forth in SEQ ID NO:1 is 34 794) and the number of matches is 33 401 , then the sequence has a percent identity of 96 (i.e., 33 401 ÷ 34 794 * 100 = 96) to the sequence set forth in SEQ ID NO:1.

The present disclosure also provides a purified and isolated DNA sequence comprising the nucleotide sequences defined by positions 30812-31789 and 18255-21101 in SEQ ID NO:1, further comprising an inserted heterologous nucleic acid fragment.

In particular, the present disclosure provides a novel adenoviral vector comprising the sequence of SEQ ID NO: 1 or a fragment thereof, further comprising one or more inserts of a heterologous nucleic acid fragment.

Further, the disclosure provides a vector as above, wherein said vector further comprises a foreign nucleic acid fragment that can express in a human a therapeutic and/or prophylactic agent or a foreign nucleic acid which in itself has a therapeutic and/or prophylactic utility.

The present disclosure further provides a method of gene therapy comprising the administration to a human, preferably a human patient, in need of such gene therapy a therapeutically effective amount of a vector as defined above.

The present disclosure further provides the use of a vector comprising a sequence of SEQ ID NO: 1 or a sequence substantially homologous therewith, further comprising one or more inserts of a heterologous nucleic acid fragment in gene therapy.

The present disclosure further provides the use of a vector comprising a sequence of SEQ ID NO: 1 or a sequence substantially homologous therewith, in gene therapy in cancer therapy. The disclosure also encompasses the use of a vector comprising a sequence of SEQ ID NO: 1 or a sequence substantially homologous therewith, in gene therapy in vascular therapy.

The present disclosure further provides use of a vector comprising a sequence of SEQ ID NO: 1 and an inserted selected therapeutic gene operatively linked to regulatory sequences which direct expression of said gene in the production of a medicament for treating a patient having an acquired or inherited genetic defect.

The disclosure makes available the use of a vector as defined above for the transfection of human cells chosen among cells of neural origin, and in particular for the infection of human cells chosen among glioblastoma, neuroblastoma and medulloblastoma, as wells haemopoietic progenitor cells.

The disclosure makes available the use of a vector as defined above for the transfection of human cells chosen among hepatoma cells, breast cancer cells, prostatic cancer cells and endothelial cells.

The disclosure makes available the use of a vector as defined above for the transfection of human cells chosen among dendritic cells.

An adenoviral vector according to the present disclosure bearing a heterologous nucleic acid fragment encoding a product that has therapeutic and/or prophylactic utility may be administered to a human or other human patient, preferably suspended in a biologically compatible solution or a pharmaceutically acceptable delivery vehicle. A suitable vehicle is sterile saline solution. Other aqueous and non-aqueous isotonic sterile injection solutions and aqueous and non-aqueous sterile suspensions known to be pharmaceutically acceptable carriers and well known to those of skill in the art may be employed for this purpose.

A composition for administration of an adenoviral vector disclosed herein may be formulated to contain other components, such as adjuvants, stabilisers, pH adjusters, preservatives and the like. Such components are well known to persons skilled in the relevant art of viral gene therapy.

The adenovirus vectors disclosed herein are administered in a pharmaceutically effective amount, i.e. an amount that is effective to transfect the desired cells--in the chosen route of administration--and provide sufficient level of expression of the selected gene to provide a therapeutic benefit.

Conventional and pharmaceutically acceptable routes of administration include, but are not limited to, intravesicular, intraductal, intranasal, intramuscular, intratracheal, subcutaneous, intradermal, rectal, oral, instillation into the urinary bladder and other parenteral routes of administration. Routes of administration may be combined, if desired, or adjusted depending upon the therapeutic goal, e.g. elicitation of immunity, and primarily on the nature of the disease being treated.

The adenovirus vectors according to the invention can be propagated and produced in any suitable cell line, such as in Vero cells from monkey kidney, A-549 cells, human embryonic retinoblast (HER) cells, such as HER 911, human embryonic kidney (HEK) cells, such as HEK 293, as well as other cell lines approved for human vaccine production such as the MRC-5, the WI-38 and the FRhL-2 cell lines.

Accordingly, the present invention further provides cells transfected with adenovirus vectors according to the invention, and methods for the production of adenovirus vectors according to the invention using such cells.

### EXAMPLES

### EXAMPLE 1. Replication-Competent Ad11p Vector (RCAdllp) Efficiently Transduces and Replicates in Hormone-Refractory Metastatic Prostate Cancer Cells

### Materials and Methods

### Cell lines and culture conditions

The human embryonic kidney cell line 293 (HEK-293), which expresses the adenoviral E1A and E1B gene products, was purchased from Microbix Biosystems (Toronto, ON, Canada). The tumor cell lines LNCaP (from prostate; metastatic site: left supraclavicular lymph node carcinoma), DU 145 (from prostate; metastatic site: brain carcinoma), and PC-3 (from prostate; metastatic site: bone adenocarcinoma) were obtained from the American Type Culture Collection (ATCC, Manassas, VA). The cell lines were all cultured at 37°C in 5% CO2, using culture medium recommended by the ATCC. The HEK-293 cell line and human A549 cell line (respiratory oat cell carcinoma) were grown at 37°C in Dulbecco's modified Eagle's medium (Sigma-Aldrich, St. Louis, MO), 20mM HEPES (pH 7.4), penicillin-streptomycin (100 IU/ml and 100 mg/ml, respectively), and with 10% fetal bovine serum (FBS) for 293 cells but only 5% for A549 cells. On viral infection, the FBS concentration was reduced to 5% for 293 cells and 2% for A549 cells. Adenoviruses and vectors Ad11 prototype (strain Slobitski) was propagated in A549 cells and purified on CsCl as described elsewhere (Mei et al., 1998). The virion band was collected. The buoyant density was checked with a refractometer and the viral concentration was measured in a spectrophotometer (Saveen & Werner, Limhamn, Sweden). The antiserum against purified virions of adenovirus type 11 was as previously described by Wadell et al. (1999. "Adenoviruses. "Manual of Clinical Microbiology. ASM Press Washington, D. C).

### RCAd11p vector

The replication-competent RCAd11p vector was constructed by insertion of an expression cassette comprising the CMV promoter, the GFP gene, and the SV40 poly(A) signal into the Ad11p genome. The insertion was made in the E1 region, at a position corresponding to nucleotide 451 in SEQ ID NO:1 Construction ofg RCAd11pGFP is described in Figure 8.

### Determination of cell surface molecules by flow cytometry

Cytometric analysis was used to determine the levels of various molecules on the cell surface. Briefly, the expression levels of CAR, CD46, CD80, CD86, α*_{ν}*β₃, and α*_{ν}*β₅ on the cells were investigated with mouse monoclonal antibodies: anti- CAR (clone RmcB; Chemicon International/Millipore, Temecula, CA) diluted 1:200, mouse anti-human CD46 monoclonal antibody (169-1-E4.3; Ancell, Bayport, MN) or rabbit antihuman CD46 polyclonal antibody (provided by J.P. Atkinson, Washington University School of Medicine, St. Louis, MO) diluted 1:200, anti-human CD80 (B7-1; Ancell) diluted 1:50, anti-human CD86 (B7-2; Ancell) diluted 1:200, anti-human integrin αᵥβ₃, (MAB1976; Chemicon International/Millipore) diluted 1:200, or anti-human integrin αᵥβ₅ (MAB1961, Chemicon International/Millipore) diluted 1:1000. Fluorescence staining of membranes was performed on a single-cell suspension by adding one of the previously mentioned monoclonal antibodies followed by fluorescein isothiocyanate (FITC)-conjugated secondary antibodies (FITC-conjugated goat anti-mouse Fab F2653; Sigma-Aldrich). Flow cytometry (FACScan; BD Biosciences, San Jose, CA) was performed at 488 nm, using LYSYS II software (BD Biosciences), by gating the distribution of the negative control sample for each individual cell line. This setting was used to determine the geometric mean value and percentage of cells expressing each of the previously mentioned markers for each individual cell line.

### Detection of virus-mediated gene delivery

A standard number of cells of each cell line (2x10⁵ cells) was used to seed each well of a 24-well dish. On the next day, the cells were transduced with 0.01, 0.05, 0.1, 0.5, or 1 pg of RCAd11pGFP vector per cell for 1 hr. Forty-eight hours later, the cells were harvested and centrifuged at 300 × g for 5 min. They were then fixed with 3ml of 2% paraformaldehyde (PFA) for 15 min at room temperature, centrifuged at 300 × g for 5 min, washed with 3ml of 2% FBS in phosphate-buffered saline (PBS), centrifuged at 300 × g, and then resuspended in 300 ml of 2% FBS in PBS (bovine serum albumin [BSA]-PBS). Finally, green fluorescent protein (GFP) expression was measured by flow cytometry. The amount of viral particles (VP) is represented by the measurement of picograms, with 1 pg representing approximately 3600 physical particles and corresponding to 50 times the 50% tissue culture infective dose (TCID₅₀), because when RCAd11pGFP vector was titered it was demonstrated that 1 TCID₅₀ unit corresponds to 72 VP. Virion concentration was determined by spectrophotometry at 260 and 330nm according to the following formula: 1 unit of absorbance at A260 - A330 is equivalent to 10¹² particles/ml or 280 mg of viral particles per milliliter.

### Antibody blocking of RCAd11pGFP-mediated transduction

The cells were grown in 12-well plates overnight and incubated with either monoclonal anti-CD46 antibody or rabbit polyclonal anti-CD46 antibody at a dilution of 1:200 in PBS for 30 min at 37°C. They were infected with virus corresponding to 0.5 pg/cell (for A549 cells) or 1 pg/cell (for 293, PC-3, DU 145, and LNCaP cells) at 37°C for 1 hr. The cells were then washed twice with BSA-PBS, and cultivated at 37°C for 48 hr. The infected cells were detached with EDTA and 0.1% trypsin in PBS and resuspended in medium with 10% FBS. They were centrifuged and resuspended at 4°C for 1 hr in 1 ml of PBS containing 2% PFA. The cells were centrifuged again and the cell pellets were resuspended in 200 ml of PBS and placed in the wells of a 96-well plate, which was kept at 4°C for 30 min. The cells were then washed with BSA-PBS and transferred in the same buffer to flow cytometry tubes.

### Assessment by flow cytometry of capacity of Ad11prfib and anti-Ad11prfib antiserum to block GFP expression

Various amounts (0.5, 3, 6, and 10 µg) of Ad11p recombinant fiber knob protein (Ad11prfib) were added to 10⁶ cells and incubated on ice for 30 min. The cells were then inoculated with RCAd11pGFP vector at 0.5 pg/cell (for A549 cells) or 1.0 pg/cell (for 293, DU 145, PC-3, and LNCaP cells) and incubated at 37°C for another 30 min. They were washed once with PBS containing 2% FBS, and then fresh culture medium was added. The number of infected cells was assessed by flow cytometry at 48 hr postinfection. The cells were seeded in 12-well plates and grown overnight. Serially diluted anti- Ad11p recombinant fiber (rfib) antiserum was premixed with RCAd11pGFP virions at 2 pg/cell for 1 hr at room temperature, and then moved to 12-well plates. Other steps were as described previously.

### Isolation of viral DNA

One million cells of each cell line were inoculated with RCAd11pGFP (0.1 pg/cell) and harvested at 24, 48, and 72 hr postinfection. Viral DNA was purified according to the method of Shinagawa and colleagues (1983). The amount of purified DNA was measured with a NanoDrop (Saveen & Werner). The DNA was digested with BamHI restriction enzyme and separated on a 1% agarose gel.

### Viral protein Western blot analysis

The five cell lines were seeded at 1.5×10⁵ cells per well and infected with RCAd11pGFP or wild-type Ad11p (Ad11pwt) at 0.1 pg/cell. At 96 hr postinfection, the cells were collected by centrifugation at 800 rpm for 5 min. The cell pellets were washed once with PBS and boiled in loading buffer. Samples of 1×10⁵ cells were loaded and separated on NuPage 12% Bis- Tris gels (Invitrogen, Carlsbad, CA) and then the gels were transferred to nitrocellulose. The viral proteins were detected with virion antibody from rabbit and horseradish peroxidase (HRP)-conjugated anti-rabbit (Dako, Glostrup, Denmark). Cytotoxicity assay To stain the cells with crystal violet, the medium was removed and the cells were fixed for 3 min in 3.7% PFA at room temperature. They were then washed with PBS and incubated for 3 min in 1% crystal violet in 70% ethanol. After staining, the cells were rinsed three times with water and air dried for photography.

### Cell proliferation assay

To evaluate the oncolytic effect of RCAd11pGFP in prostate cancer cells, cells were infected with RCAd11pGFP and Ad11pwt at 10⁻⁴, 10⁻³, 10⁻², 10⁻¹, and 10° viral pg per cell, corresponding to 0.36, 3.6, 36, 360, and 3600 VP/cell. Cell viability was determined by XTT (sodium 3-[1 -(phenylaminocarbonyl)- 3,4-tetrazolium] -bis(4-methoxy-6-nitro)benzene sulfonic acid hydrate) assay at 6 days postinfection (cell proliferation kit II; Roche Applied Science, Indianapolis, IN). Quantitation was performed colorimetrically at a wavelength of 490 nm.

### One-step growth curves of Ad11pwt and RCAd11pGFP

Monolayers of DU145, PC-3, andLNCaP cells were infected with either RCAd11pGFP or wild-type Ad11 (Ad11pwt) at 0.2 pg/cell. At 24, 48, 72, and 96 hr postinfection, duplicate cell samples were harvested and lysed by three cycles of freeze-thawing, and the virus in the supernatants was assayed in HEK-293 cell monolayers. One-step growth curves of the viruses were performed in LNCaP cells withmethyltrienolone (R1881) in the medium. Viral yields were titrated 96 hr after infection. Duplicate sets of cells were infected, and the assays were carried out four times. A classical TCID₅₀ assay was used to measure the viral titration.

### Animal experiments

BALB/c nude mice were obtained from Taconic (Ry, Denmark) at 3-4 weeks of age and they were quarantined for at least 1 week before study. PC-3 cells were grown in F12K medium with 10% FBS, 2mM 1-glutamine, and penicillin and streptomycin, until the cells were confluent. They were harvested by two consecutive trypsinizations, centrifuged at 300×g for 5 min, washed twice, and resuspended in PBS, and the cell density was adjusted to 5×10⁷ cells/ml. Then 1×10⁷ cells in 0.2 ml were injected subcutaneously into each nude mouse. When tumors with a volume of 75mm³ developed animals were randomly assigned to three groups: two treated groups received one intratumoral injection of 50 mg of RCAd11p vector or Ad11pwt virus that was diluted in a volume of 100 ml of PBS; one untreated group received PBS only. Tumor axes were measured weekly with vernier calipers, and tumor volume was calculated according to the following simplified formula: (l×w×d)/2.

### Statistical analysis

Statistical analyses (t tests) were performed with GraphPad Prism software version 4.03 (GraphPad Software, San Diego, CA).

### Results

### CD46 and CAR show different expression patterns on the surface of prostate metastatic carcinoma cell lines

The distribution of cell surface molecules that function as receptors or potential receptors for Ad11 vector in prostate and control cell lines was studied. CAR and CD46 serve as receptors for Ad5 and Ad11, respectively (Tomko et al. 1997. HCAR and MCAR: the human and mouse cellular receptors for subgroup C adenoviruses and group B coxsackieviruses. Proc Natl Acad Sci U S A 94(7), 3352-6; Gaggar et al. 2003. CD46 is a cellular receptor for group B adenoviruses. Nat Med 9(11), 1408-12.; Segerman et al. 2003. Adenovirus type 11 uses CD46 as a cellular receptor. J Virol 77(17), 9183-91). Cytometric analysis of the immunofluorescence staining of three human prostate cancer cell lines (PC-3, DU 145, and LNCaP) and control cell lines (A549 and HEK-293) showed that the highly tumorigenic cell line PC-3 had a geometric mean value of 95.8 for CD46 expression and only 2.9 for CAR-positive cells. DU 145 manifested the highest amount (198.5) of CD46 expression whereas LNCaP cells had a relatively lower number (44.9) of CD46-positive cells. The geometric mean values for CAR expression in DU 145 and LNCaP cells were 31.2 and 65.6, respectively. A549 and 293 cells derived from lung and kidney had clear expression of CAR (56.3 and 59.0). CD46 showed different expression patterns in these two cell lines: 71.9 for A549 cells and 50.6 for 293 cells. Consequently, DU 145 showed a high degree of expression of CD46 (198.5) as compared with the other cell lines studied, whereas PC-3 cells showed minimal expression of CAR (Figure 1A).

To verify that interaction of the penton base of RCAd11pGFP vector with cells was also mediated by integrins α*_{ν}*β₃ and α*_{ν}*β₅ the amounts of these molecules on the prostate cancer cell surface were compared with the amounts on A549 and HEK-293 cells. LNCaP cells were cultivated in medium containing R1881 to maintain the androgen dependent property. α*_{ν}*β₃ and integrins were detected at low geometric mean values (2.6 and 2.9, respectively) in LNCaP cells. However, in both androgen-independent PC-3 and DU 145 cell lines, the sum of α*_{ν}*β₃ and α*_{ν}*β₅, integrins was expressed to similar extents: 4.8 and 18.9 for PC-3 cells, and 5.2 and 15.5 for DU 145 cells, respectively (Figure 1A).

CD80 and CD86 are widely distributed on the surfaces of blood cells and endothelial cells. They are costimulatory molecules involved in T cell activation. It has been reported that all species B adenoviruses use CD80 and CD86 for primary attachment to cells (Short et al. 2006. Members of adenovirus species B utilize CD80 and CD86 as cellular attachment receptors. Virus Res 122(1-2), 144-53). Ad11 of species B:2 has two receptors: CD46 and unknown receptors. Thus, to investigate the possibility and to confirm that either CD80 or CD86 also acts as a receptor for Ad11, these two molecules were included in the receptor screen for Ad11 virus. The expression of CD80 and CD86 was barely detected in all cell lines, with the exception of HEK-293 and DU 145 cells, which exhibited small amounts of CD80 (2.78 and 3.5%) and CD86 (1.62 and 2.5%), respectively. Taken together, these results indicate that CD80 and CD86 might also serve as receptors for Ad11-mediated gene transfer in metastatic prostate cancer cells (Figure IB).

### The transduction efficiency of RCAd11pGFP is proportional to the level of available CD46 molecules and integrins

Previous studies have documented the significance of the higher binding activity of Ad11 virus to prostate metastatic cell lines (Zhang et al. 2003. Human adenovirus serotypes 4 and 11 show higher binding affinity and infectivity for endothelial and carcinoma cell lines than serotype 5. J Gen Virol 84(Pt 3), 687-95). To determine whether there was a correlation between viral binding and the number of cell surface molecules that are putative or established primary or secondary receptors, and the extent of correlation with gene transduction efficacy, the three human prostate metastatic cell lines (PC-3, DU 145, and LNCaP cells) and the two control cell lines (A549 and HEK-293) were compared. An RCAd11pGFP vector in which expression of GFP is driven by the cytomegalovirus (CMV) minimal promoter was used. To quantify GFP expression in various prostate carcinoma cells, RCAd11pGFP virus at 0.01, 0.05, 0.1, 0.5, and 1 pg/cell (corresponding to 36, 180, 360, 1800, and 3600 VP/cell or 0.5, 2.5, 5, 25, and 50 TCID₅₀, respectively) was used to infect the various prostate carcinoma cells and flow cytometric assays were used to monitor the levels of expression of GFP. RCAd11pGFP vector was capable of infecting all cell lines tested (Figure 2), but significantly different levels of GFP expression were observed in cells infected with a low dose of RCAd11pGFP. With RCAd11pGFP at 0.01 and 0.1 pg/cell, less than 5% of the LNCaP cells expressed GFP whereas 10-40% of A549, HEK- 293, and PC-3 cells expressed GFP. After infection with the same dose of RCAd11pGFP, DU 145 cells showed higher GFP expression than did LNCaP cells (Figure 2).

LNCaP cells, which were less efficiently infected with RCAd11pGFP, also expressed CD46, αᵥβ₃ and αᵥβ₅ integrins at lower levels than did PC-3 and DU 145 cells. DU 145 cells were more prone to grow in clusters than the other cells, which might reduce the opportunity of RCAd11pGFP virions to reach all the DU 145 cells.

### Transduction is reduced by anti-CD46 antibodies, Ad11prfib, as well as antibodies to Ad11prfib, and integrins

To investigate the importance of CD46 as a primary receptor for RCAd11pGFP vector transduction, blocking experiments with polyclonal and monoclonal anti- CD46 antibodies were performed. The efficiency of infection was assessed by quantification of GFP expression by flow cytometric assay. As shown in Figure 3A and 3B, monoclonal anti-CD46 antibody (clone E4.3) was able to inhibit 5% (HEK-293) to 15% of GFP-positive cells, whereas polyclonal anti-CD46 antibody reduced the expression of GFP up to 20% in all cell lines studied. In general, polyclonal anti-CD46 antibody gave stronger inhibition than the monoclonal anti-CD46. GFP expression in PC-3 cells was inhibited approximate 20% when using polyclonal anti-CD46 antibody.

Ad11prfib effectively inhibited transduction mediated by the RCAd11pGFP vector. With Ad11prfib at 0.5 pg/cell, transduction was inhibited 20% in HEK-293 cells and 50% in A549 and DU 145 cells (Figure 3C). With Ad11prfib at 10 pg/cell, GFP expression could be blocked to 5% in PC-3 and DU 145 cells or 10% in LNCaP, HEK-293, and A549 cells. In addition, the ability to inhibit RCAd11pGFP infection with anti- Ad11prfib antiserum was remarkably efficient in comparison with the results of the CD46 antibody blocking assay (Figure 3D).

After blocking with anti-integrin antibodies, the extent of GFP expression varied in the various cell lines; however, in general, anti-α*_{ν}*β₃ antibody blocked uptake of virions in all cell lines effectively, whereas anti-α*_{ν}*β₅ antibody inhibited GFP expression in all lines with the exception of A549 cells. This is in line with the high expression of integrin (Figure 3E). Thus, CD46 may not be the only primary receptor for Ad11 when infecting human prostate cancer cells. Both integrin α*_{ν}*β₃ and integrin α*_{ν}*β₅ affected transduction mediated by the RCAd11pGFP vector.

### RCAd11p replication and induction of cytopathic effect do not correlate with cellular p53 status

Early studies by Hall and colleagues suggested that Ad5 replication required cellular p53 status in the infected cells. This report was not consistent with the later results from Harada and Berk, who claimed that cytopathic effect induced by Ad5 was independent of cellular p53 status. However, there are no reports concerning Ad11 viral replication and cellular p53 level. Therefore, it was investigated whether p53- dependent cell death was needed for induction of RCAd11p cytopathic effect and release of progeny virions from infected cells. Tumor suppressor protein p53 expression in these five cell lines differs greatly: no detectable p53 in PC-3, mutant p53 (p53mut) in DU 145 and LNCaP cells, and wild-type p53 (p53wt) in A549 and HEK-293 cells have been reported (Graham et al. 1977. Characteristics of a human cell line transformed by DNA from human adenovirus type 5. J Gen Virol 36(1), 59-74; Carroll et al. 1993. p53 oncogene mutations in three human prostate cancer cell lines. Prostate 23(2), 123-34; Lehman et al. 1991. p53 mutations, ras mutations, and p53-heat shock 70 protein complexes in human lung carcinoma cell lines. Cancer Res 51(15), 4090-6; van Bokhoven et al. 2003. Molecular characterization of human prostate carcinoma cell lines. Prostate 57(3), 205-25).

The five cell lines were infected with RCAd11pGFP at 0.1 or 1 pg/cell, corresponding to 5 or 50 TCID50, respectively, and exhibited by phase-contrast microscopy 48 hr postinfection. p53-positive A549 cells and p53-negative PC-3 cells showed obvious cytopathic effect (CPE) by 48 hr at 0.1 pg/cell whereas p53-positive HEK-293 cells and p53-mutant DU 145 and LNCaP cells exhibited CPE with 1 pg/cell by this time (Figure 4A). Thus, RCAd11pGFP induction of CPE in various cell lines varies widely and dose not correlate with the expression of wild-type p53.

The replication properties of the RCAd11pGFP vector were investigated in prostate cancer cells, and in A549 and HEK- 293 cells. The cells were infected with 0.1 pg/cell, and then viral DNA was extracted. Good yields of viral DNA were obtained in PC-3 cells, similar to those in A549 and HEK-293 cells, but the viral DNA was detectable in DU 145 and LNCaP cells at 1 day postinfection. At 2 days postinfection, the level of viral DNA was increased to a high level in PC-3 and DU 145 cells as well as in the control cells (Figure 4B and 4C). There was also an increased DNA concentration in LNCaP cells, but the yield was lower than in other cells. The amount of the characteristic restriction fragments revealed that the vector DNA replicated in PC-3 cells as efficiently as in A549 cells during the observation period. Late expression of viral proteins in the prostate cancer cell lines was also compared and it was found that the yield of hexons correlated with the cytotoxicity results: hexon protein was highly expressed in PC-3 cells but was just detectable in LNCaP cells. An intermediate amount of hexons was observed in DU 145 cells (Table 1). Therefore, DNA replication, protein expression, and oncolytic effects of RCAd11pGFP were not dependent on the p53 level of the target cells but, rather, on the intrinsic properties of targeting tumor cells.

**Table 1. Transduction and cytolytic effect mediated by RCAd11pGFP are independent of p53 level in targeting cell lines.**

| Cell type | p53 gene status*^{a}* | % CD46 expression | % CAR expression | %GFP expression at 1pg/cell, 48h p.i. | DNA replication (ng /10⁴ cells)*^{b}* | CPE with 0.1 pg/cell at 48 h p.i. | Viral protein level*^{c}* | Cytotoxic assay at day 6 p.i. pg/cell | One step replication | |
|---|---|---|---|---|---|---|---|---|---|---|
| | | | | | | | | | Day 1 | Day2 |
| A549 | p53wt | 90.1 | 80.3 | 85 | 113.5 | ++++ | ++++ | 0.0001 | 10⁶ | 10⁸ |
| HEK293 | p53wt | 67.1 | 88.3 | 70 | 60 | ++ | +++ | 0.001 | 5x10⁵ | 5x10⁷ |
| PC-3 | p53neg | 87.4 | 4.7 | 80 | 110 | ++++ | ++++ | 0.0001 | 10⁶ | 10⁸ |
| DU145 | p53mut | 94.1 | 64.3 | 67 | 50 | ++ | +++ | 0.01 | 5x10⁵ | 5x10⁷ |
| LNCaP | p53mut | 72.4 | 82.7 | 50 | 48 | ++ | ++ | 0.01 | 10⁴ | 10⁶ |

| | | | | | | | | | | |
|---|---|---|---|---|---|---|---|---|---|---|
| Abbreviations: CAR, coxsackievirus-adenovirus receptor; CPE, cytopathic effect; GFP, green fluorescent protein; p53mut, mutant p53; p53neg, negative for p53; p53wt, wild-type p53; p.i., postinfection. ^{a}p53 gene status was determined from the literature: Carroll et al., 1993; Hall et al., 1998; Lehman et al., 1991; van Bokhoven et al., 2003. ^{b}Qualitative assessment of viral DNA isolated from RCAd11pGFP vector at 72 hr postinfection. ^{c}Determined by Western blot analysis of viral protein expression levels | | | | | | | | | | |

### RCAd11pGFP vector can spread efficiently from cell to cell in p53⁻ PC-3 and p53⁺ A549 cells

To achieve oncolytic efficacy, a replication-competent tumor therapy vector should be able to spread rapidly from tumor cell to tumor cell. The high expression of GFP mediated by the RCAd11pGFP vector should lead to rapid cell lysis and vector release, resulting in an increasing infection-release- reinfection cycle. To evaluate the likelihood of this reasoning, a toxicity assay on prostate cancer cells was performed, A549 cells, and HEK-293 cells infected with RCAd11pGFP and wild-type Ad11p. As shown in Figure 5A and Table 1, cells in 24-well plates were infected with four 10-fold dilutions of the two viruses, starting at 1 pg/cell. The negative control had no virus added. At 6 days postinfection, the monolayers were stained with crystal violet as described in Materials and Methods. Cells infected at the highest multiplicity of infection (1 pg/cell) were rapidly destroyed. However, in cells infected with 0.0001 pg/cell, the virus must initiate one or more cycles of infection to cause detectable cytopathic effect. RCAd11pGFP replicated 2 logs more efficiently in PC-3 cells than in LNCaP cells. These data indicate that of the five cell lines tested, the most efficient growth of RCAd11pGFP was in PC-3 and A549 cells (Table 1). Furthermore, no great difference in the growth patterns of RCAd11pGFP and wild-type Ad11p was observed (Figure 5A).

### RCAd11pGFP significantly reduces proliferation of PC-3 and DU 145 cells, but not that of LNCaP cells

To study the effect of RCAd11p vector and Ad11pwt on cell viability and proliferation, the three prostate metastatic cell lines were infected with the viruses. As shown in Figure 5B, viable cells and proliferation of PC-3, DU 145, LNCaP cells were obviously reduced at 3.6, 36, and 360 VP/cell, respectively. No distinct difference between RCAd11pGFP and Ad11pwt was observed in the cell viability assay. PC-3 and DU 145 cells were more sensitive to RCAd11pGFP infection than were LNCaP cells. The strongest effect was observed at 1 pg/cell (3600 VP/cell), which caused the death of more than 90% of PC-3 and DU 145 cells and 80% of LNCaP cells at 6 days postinfection.

### RCAd11pGFP vector multiplies 100 times more efficiently in PC-3 cells than in LNCaP cells

The replicative capacity of RCAd11pGFP was determined by a one-step growth assay. Cells were infected with 0.2 pg of virus per cell, and the total amount of vector was harvested 24, 48, 72, and 96 hr postinfection and measured by titration in HEK-293 cells. The viral titer was higher in PC-3 cells than in the other two cell lines; it was 5 logs higher after 96 hr of incubation. In DU 145 cells it was slightly less over the same time period, reaching 4.5 logs. In contrast, the viral titer of vector in LNCaP cells was 3 logs higher than in the original inoculum. Thus, RCAd11p vector did grow 100-fold better in PC-3 cells than in LNCaP cells. With the same infectious dose and the same incubation period, the different capacity of RCAd11pGFP to grow in prostate metastatic cells suggested that the natural property of the vector and the tumorigenicity of targeting cells are decisively important for viral replication (Figure 5C).

### Antitumor effect of RCAd11p vector in PC-3 subcutaneous tumor

The weight of each animal and the size and shape of tumors were measured and photographed before injection with virus and before euthanasia. Athymic BALB/c mice 4-5 weeks of age with PC-3 subcutaneous tumor were treated with either RCAd11p vector or Ad11pwt virus once via intratumoral injection. Control group tumors were treated with PBS. All inoculated or control mice survived the 6-week experimental period. After treatment, the group treated with RCAd11p vector or Ad11pwt virus demonstrated significant growth inhibition of the PC-3 tumors for up to 42 days as compared with mock-treated PC-3 tumors (Figure 6A and 6B); which were 2.5- to 3.5-fold greater in size, respectively, than the tumors treated with RCAd11p or Ad11pwt.

### Discussion

The lethal phenotypes of human prostate cancer are characterized by progression from androgen dependence to androgen independence, with a propensity to form bone metastases. In almost 80% of prostate cancer cases, the bone is colonized and a characteristic osteoblast reaction is elicited. Despite therapeutic advances involving combinations of chemotherapy, radiation treatment, and immunotherapy, most patients with metastatic prostate cancer still die within a few years (Hickey et al. 1988. Failure of chemotherapy to prolong life in patients with metastatic prostate cancer who have failed androgen deprivation. Urology 31(1), 38-40; Coleman, 2006. Clinical features of metastatic bone disease and risk of skeletal morbidity. Clin Cancer Res 12(20 Pt 2), 6243s-6249s; Mike et al. 2006. Chemotherapy for hormone-refractory prostate cancer. Cochrane Database Syst Rev (4), CD005247). Gene therapy mediated by Ad5 has shown less efficient transduction of metastatic prostate carcinoma and clinical bladder cancer because these cells lack the Ad5 primary receptor (CAR) (Li et al. 1999. Loss of adenoviral receptor expression in human bladder cancer cells: a potential impact on the efficacy of gene therapy. Cancer Res 59(2), 325-30; Rauen et al. 2002. Expression of the coxsackie adenovirus receptor in normal prostate and in primary and metastatic prostate carcinoma: potential relevance to gene therapy. Cancer Res 62(13), 3812-8; Sachs et al. 2002. Integrin alpha(v) and coxsackie adenovirus receptor expression in clinical bladder cancer. Urology 60(3), 531-6.). There is increasing awareness that the development of novel alternative adenoviral vectors that target non-CAR receptors with low seroprevalence may improve survival rates among patients with this form of cancer. To address this end, a genetically engineered RCAd11p vector was assessed and it was explored whether this virus could be efficiently expressed in cell lines derived from metastatic prostate cancer.

The present inventors have described *in vitro* and *in vivo* characterization of a novel replication-competent Ad11 vector that expresses the entire viral genome and, in addition, a GFP expression cassette. This vector contains both the E1A and E1B 55k open reading frames (ORFs). Ad5 vector with the E1B55k ORF deleted has been found to show tumor-specific replication (Bischoff et al. 1996. An adenovirus mutant that replicates selectively in p53-deficient human tumor cells. Science 274(5286), 373-6). However, in the latter report there is no information given about the efficiency of viral replication in the presence of the E1B55k ORF. The role of the E1A and E1B ORFs were taken into account and these ORFs were retained in the RCAd11p vector, with the purpose of investigating the natural properties of the vector, such as transduction and oncolysis.

Integrins α*_{ν}*β₃ and α*_{ν}*β₅ are secondary receptors for Ad2 and Ad5 (Wickham et al. 1993. Integrins alpha ν beta 3 and alpha ν beta 5 promote adenovirus internalization but not virus attachment. Cell 73(2), 309-19). α*_{ν}*β₃ and α*_{ν}*β₅ integrins apparently showed higher expression in PC-3 and DU 145 cells than in LNCaP cells. With monoclonal antibody to these integrins (MAB1976) recognizing the vitronectin receptor in α*_{ν}*β₃ complex, an RGD-directed adhesion receptor, transduction mediated by the Ad11 vector was reduced by 10 and 40% for A549 and PC-3 cells, respectively. The presently results suggest that these integrins play an important role in infection of metastatic prostate cancer cells by Ad11.

Thus, transduction by RCAd11pGFP relies on both CD46 and integrins on prostate cancer cells. With high amounts of CD46 and integrins on PC- 3 and DU 145 cells (Table 1), GFP expression was higher in these cells than in LNCaP cells, which appear to express relatively low numbers of such receptor molecules. The transduction efficiency was also dose dependent, as with other viral vectors (Kustikova et al. 2003. Dose finding with retroviral vectors: correlation of retroviral vector copy numbers in single cells with gene transfer efficiency in a cell population. Blood 102(12), 3934-7).

The CD46 molecule consists of four domains: CCP1, CCP2; CCP3, and CCP4 (also designated SCR1, SCR2, SCR3, and SCR4). It was previously demonstrated that Ad5/Ad35 chimeric virus requires CCP2 (SCR2) for infection (Gaggar et al. 2005. Localization of regions in CD46 that interact with adenovirus. J Virol 79(12), 7503-13; Stone et al. 2005. Development and assessment of human adenovirus type 11 as a gene transfer vector. J Virol 79(8), 5090-104). In this study, the CD46-specific mAb (clone E4.3), which recognizes the SCR1 domain, only partially blocked the attachment of Ad11 virions to prostate cancer cells. Some epitopes recognized by other anti-CD46 mAbs (MEM258 and M177) can more efficiently block Ad35 binding to the cell surface, as reported by Fleischli et al. (2005. The distal short consensus repeats 1 and 2 of the membrane cofactor protein CD46 and their distance from the cell membrane determine productive entry of species B adenovirus serotype 35. J Virol 79(15), 10013-22). Furthermore, the crystal structure of the complex between Ad11prfib and CD46 has been elucidated, and this indicated that Ad11prfib interacts with both the SCR1 and SCR2 domains of the CD46 molecule (Persson et al. 2007. Adenovirus type 11 binding alters the conformation of its receptor CD46. Nat Struct Mol Biol 14(2), 164-6). Ad11 binding reshapes the conformation of its receptor so that the bent surface structure straightens into an elongated rod. All these results might explain why neither anti-CD46 mAb nor polyclonal antibodies can efficiently block viral infection. The fiber is the main viral ligand for attachment to prostate cancer cell lines, because viral infectivity was almost totally inhibited by Ad11prfib or anti-rfib antibody.

The three classic prostate cancer cell lines express different amounts of tumor suppressor protein (p53). PC-3 cells do not express any detectable p53, whereas LNCaP cells and DU 145 cells produce mutant p53 (Carroll et al. 1993. p53 oncogene mutations in three human prostate cancer cell lines. Prostate 23(2), 123-34; van Bokhoven et al. 2003. Molecular characterization of human prostate carcinoma cell lines. Prostate 57(3), 205-25). PC-3 cells are derived from a highly tumorigenic metastatic prostate carcinoma.

Both the PC-3 and the DU 145 cell lines are androgen independent whereas LNCaP cells are androgen-dependent metastatic cells. Regarding clinical treatment, androgendependent prostate cancer is sensitive to chemotherapy. When prostate cancer cells develop androgen independence, all kinds of treatment become ineffective. Interestingly, the replicative capacity of Ad11 in PC-3 and DU145 cells was 100- fold more efficient than in LNCaP cells (Table 1).

The results present here demonstrate that the RCAd11 vector may be a promising tool for the treatment of metastatic prostate cancer. Early reports suggested that ONYX-015 (also called d11520), based on the Ad5 vector with deletion of the E1B 55k ORF, did selectively kill tumor cells harboring a defective p53 gene (Bischoff et al. 1996. An adenovirus mutant that replicates selectively in p53-deficient human tumor cells. Science 274(5286), 373-6.; Heise et al. 1997. ONYX-015, an E1B gene-attenuated adenovirus, causes tumor-specific cytolysis and antitumoral efficacy that can be augmented by standard chemotherapeutic agents. Nat Med 3(6), 639-45); however, subsequent reports have shown that ONYX-015 replicates and kills tumor cells with wild-type p53 at least as efficiently as it kills cells lacking p53 (Hall et al. 1998. p53-dependent cell death/apoptosis is required for a productive adenovirus infection. Nat Med 4(9), 1068-7; Rothmann et al. 1998. Replication of ONYX-015, a potential anticancer adenovirus, is independent of p53 status in tumor cells. J Virol 72(12), 9470-8; Dix et al. 2000. Efficient induction of cell death by adenoviruses requires binding of E1B55k and p53. Cancer Res 60(10), 2666-72).

The present results based on an RCAd11p vector, demonstrate that Ad11 vector carrying the E1B 55k ORF replicates efficiently in tumor cells expressing wild-type p53 and expressing mutant p53 as well as lacking p53 expression.

Previously it was reported that replication of the Ad5 wildtype or dl1520 strain was 4-to 24-fold lower in A549 than in PC-3 cells (Harada and Berk, 1999. p53-Independent and - dependent requirements for E1B-55K in adenovirus type 5 replication. J Virol 73(7), 5333-44). Low expression of CAR in the highly tumorigenic PC-3 cell line was observed in this study, suggesting that the CAR-targeting vector, Ad5, might not be suitable for gene therapy in such tumor types. CD46 was highly expressed on the surface of PC-3 cells and RCAd11pGFP infected PC-3 cells easily and caused high expression of the GFP gene. It has previously been reported that wildtype Ad11p shows higher binding affinity for two prostate cell lines than does Ad5 (Zhang et al. 2003. Human adenovirus serotypes 4 and 11 show higher binding affinity and infectivity for endothelial and carcinoma cell lines than serotype 5. J Gen Virol 84(Pt 3), 687-95). In the present study, RCAd11p clearly showed higher infectivity in prostate cancer cells, all of which express CD46 at high levels on their surface. The CD46 molecule is widely distributed on the cell surface of nucleated human cells (Liszewski et al. 2005. Emerging roles and new functions of CD46. Springer Semin Immunopathol 27(3), 345-58.). The transduction assay mediated by interaction between RCAd11p vector and CD46 showed that this vector efficiently binds to cells, is taken up by them, and enters the nucleus.

Strains of the Edmonston lineage of measles virus also use CD46 as primary cellular receptor. The killing of tumor cells by measles virus has been found to be dependent on the density of CD46 on the cell surface (Anderson et al. 2004. High CD46 receptor density determines preferential killing of tumor cells by oncolytic measles virus. Cancer Res 64(14), 4919-26). The novel Ad11 vector showed a different way of killing target cells: first, viral transduction efficiency relied on the density of cell surface receptors, where the amount of GFP expression was correlated with the number of CD46 molecules on the cell surface; second, the oncolytic function of the vector was dependent on tumor cells and the vector construct. Not one of the two factors is dispensed to complete the oncolytic infection cycle. In the highly tumorigenic PC-3 cells, RCAd11p vector showed a high degree of replication, whereas in DU 145 cells the replicative efficacy of RCAd11p vector was better than in androgen-dependent LNCaP cells. Gene transfer to LNCaP cells mediated by the Ad11p vector was as efficient as for other cells, but the production of oncolytic vector particles is limited in LNCaP cells. Therefore, an Ad11 vector that carries a virus-specific promoter and a GFP expression cassette was generated. With this construct, it was possible to evaluate the transduction and replicative capacity of this vector. The benefit of using RCAd11p vector is that this virus vector allows efficient transduction and has a high replicative capacity in most tumorigenic cells.

Here it is demonstrated those metastatic prostate carcinoma cell lines support RCAd11pGFP replication and the production of infectious progeny, with yields from 6 to 8 logs. It is also demonstrated that RCAd11pGFP replicates in PC-3 cells most efficiently.. The PC-3 cell line was highly tumorigenic, androgen independent, and tolerant of chemotherapy.. It is further demonstrated that the oncolytic RCAd11p vector exhibits considerable antitumor efficacy in xenografted nude mice. The growth of PC-3 tumors was limited by injection of either RCAd11p or Ad11pwt, whereas control tumors without virotherapy grew rapidly. In a comparison of treated and untreated tumors, a significant difference (p< 0.001) was observed at 3 or 4 weeks postinfection. Moreover, it is demonstrated that the mice subjected to virotherapy were healthy whereas the untreated mice clearly suffered from the effect of tumors. The results demonstrate that the RCAd11p vector enhanced in vivo production of virus in initially infected tumor cells with subsequent dissemination to neighboring tumor cells. Therefore, RCAd11p efficiently exerts an oncolytic effect in xenografted nude mice.

In summary, the RCAd11p vector provides advantages for gene therapy of both androgen-independent and androgen-dependent late-stage metastatic prostate cancer, because this vector transfers genes efficiently to several human cell lines but shows high-level replication in metastatic prostate cells in bone. The oncolytic capacity of the RCAd11p vector relies on the type of tumor rather than on the presence or absence of the EIB55k ORF in the vector. These findings indicate that the RCAd11 vector-particularly under the control of the viral early region 1 (E1A) promoter-has potential for the treatment of highly tumorigenic cells. Consequently, gene therapy based on RCAd11p may be useful in the future treatment of metastatic prostate carcinomas.

### EXAMPLE 2. Effect of Replication-Competent Ad11p Vector on the growth of human colon T84 tumours in nude mice

### In vivo oncolytic model with RCAd11p vector.

T84 cells derived from a lung metastasis of a colorectal adenocarcinoma and HT 29 derived from colorectal adenocarcinoma were used in the experiment. 10⁷ T84 cells or H-29 cells in 0.2 ml were subcutaneously transplanted into left and right flank region of *Balb*/*c* nude mice, the control group mice were injected with only PBS. As colon tumor grew up to at least 75 mm³ 3 weeks after injection, 50 µg of RCAd11pGFP/tumor was injected into mice with intratumoral administration. Tumor volume was recorded weekly. The mice were sacrificed approximately 6 weeks after viral injection.

As can be seen from the results presented in Figure 10, treatment with the adenovirus vector RCAd11pGFP reduced the growth of the transplanted colon tumours to a similar extent as treatment with wild-type Ad11p, demonstrating that the recombinant adenovirus vector carrying an insertion of a heterologous nucleic acid fragment encoding GFP maintained replication competence, infectivity and oncolytic activity..

## Claims

1. A recombinant replication-competent Ad11p adenovirus vector, that comprises an isolated nucleic acid sequence having at least 90%, preferably at least 95%, more preferably at least 98%, and most preferably at least 99% sequence identity to the sequence set forth in SEQ ID NO: 1, and further comprising one or more inserts of a heterologous nucleic acid fragment(s) that is/are (i) either cloned between nucleotide 247 and nucleotide 568 of SEQ ID NO: 1 of such a vector and which vector expresses the entire viral genome or (ii) wherein said heterologous nucleic acid fragment replaces the sequence between nucleotide 28356 and 29482 of SEQ ID NO: 1 in said vector.

2. A recombinant replication-competent Ad11p adenovirus vector according to claim 1, wherein said insert has a size of between 100 and 5,000 base pairs, such as a between 500 to 2,000 base pairs.

3. A recombinant replication-competent Ad11p adenovirus vector according to any of claims 1 to 2, wherein said insert encodes a siRNA, a shRNA, or a micro RNA.

4. A recombinant replication-competent Ad11p adenovirus vector according to any of claims 1 to 3, wherein said heterologous nucleic acid fragment encodes a protein selected from the group consisting of: a viral protein, an antigenic determinant of a pathogenic organism, a tumor-specific antigen, a human protein, a cytokine.

5. A recombinant replication-competent Ad11p adenovirus vector according to any of claims 1 to 4, wherein said heterologous nucleic acid fragment encodes a reporter protein selected from the group consisting of: a fluorescent protein, a luminescent protein, and an enzyme.

6. A pharmaceutical composition comprising a recombinant replication-competent adenovirus vector according to any of claims 1 to 5, and a suitable excipient.

7. A pharmaceutical composition according to claim 6 formulated for local administration to a tumour site, for intravesical or intraductal administration, or intravenous injection.

8. A recombinant replication-competent adenovirus vector according to any of claims 1 to 5 for use as a medicament.

9. A recombinant replication-competent adenovirus vector according to any of claims 1 to 5 for use in treatment of a disease selected from the group consisting of: a viral infectious disease, a bacterial infectious disease, a cancer disease, vascular disease, a cardiovascular disease, an immunological disease, a liver disease, and a parasitic disease.

10. A recombinant replication-competent adenovirus vector for use according to claim 9, wherein said viral infectious disease is selected from the group consisting of viral diseases caused by HCV, HBV, HIV, and influenza A virus.

11. A recombinant replication-competent adenovirus vector for use according to claim 9, wherein said bacterial infectious disease is selected from the group consisting of tuberculosis, and septic shock.

12. A recombinant replication-competent adenovirus vector for use according to claim 9, wherein said cancer disease is selected from the group consisting of: lung cancer, breast cancer, ovarian cancer, prostate cancer, colorectal cancer, bladder cancer, kidney cancer, haematological malignancies, liver cancer, pancreatic cancer, tumours of neural origin, head and neck cancer, and osteosarcoma.

13. A recombinant replication competent adenovirus vector according to any of claims 1 to 5 for use in gene therapy.

14. A mammalian cell comprising a recombinant replication competent adenovirus vector according to any of claims 1 to 5.

15. A method of expressing a protein or gene product or an expression product which comprises infecting or transfecting a cell in vitro with a recombinant replication competent adenovirus vector according to any of claims 1 to 5 and optionally extracting, purifying or isolating the protein, gene product or expression product from the cell.

16. Use of a recombinant replication competent adenovirus vector according to any of claims 1 to 5 for the in vitro identification of an anti-viral compound.

## Patentansprüche

1. Rekombinanter, replikationskompetenter Ad11p-Adenovirus-Vektor, der eine isolierte Nukleinsäuresequenz mit mindestens 90 %, vorzugsweise mindestens 95 %, mehr bevorzugt mindestens 98 % und am meisten bevorzugt mindestens 99 % Sequenzidentität mit der in SEQ ID NR: 1 dargelegten Sequenz umfasst, und des Weiteren ein oder mehrere Inserte von einem oder mehreren heterologen Nukleinsäurefragment(en) umfasst, das/die (i) entweder zwischen Nukleotid 247 und Nukleotid 568 von SEQ ID NR: 1 eines solchen Vektors kloniert ist/sind und wobei der Vektor das gesamte virale Genom exprimiert, oder (ii) wobei dieses heterologe Nukleinsäurefragment die Sequenz zwischen Nukleotid 28356 und 29482 von SEQ ID NR: 1 in diesem Vektor ersetzt.

2. Rekombinanter, replikationskompetenter Ad11p-Adenovirus-Vektor nach Anspruch 1, wobei das Insert eine Größe zwischen 100 und 5.000 Basenpaaren, wie etwa zwischen 500 bis 2.000 Basenpaaren aufweist.

3. Rekombinanter, replikationskompetenter Ad11p-Adenovirus-Vektor nach einem der Ansprüche 1 bis 2, wobei das Insert für eine siRNA, eine shRNA oder eine Mikro-RNA kodiert.

4. Rekombinanter, replikationskompetenter Ad11p-Adenovirus-Vektor nach einem der Ansprüche 1 bis 3, wobei das heterologe Nukleinsäurefragment für ein Protein kodiert, das aus der Gruppe bestehend aus einem Virus-Protein, einer Antigen-Determinante eines pathogenen Organismus, einem tumorspezifischen Antigen, einem menschlichen Protein und einem Zytokin ausgewählt ist.

5. Rekombinanter, replikationskompetenter Ad11p-Adenovirus-Vektor nach einem der Ansprüche 1 bis 4, wobei das heterologe Nukleinsäurefragment für ein Reporterprotein kodiert, das aus der Gruppe bestehend aus einem Fluoreszenz-Protein, einem Lumineszenz-Protein und einem Enzym ausgewählt ist.

6. Pharmazeutische Zusammensetzung, umfassend einen rekombinanten, replikati-onskompetenten Adenovirus-Vektor nach einem der Ansprüche 1 bis 5 und einen geeigneten Hilfsstoff.

7. Pharmazeutische Zusammensetzung nach Anspruch 6, formuliert für eine lokale Verabreichung an einen Tumorort, für eine intravesikale oder intraduktale Verabreichung oder eine intravenöse Injektion.

8. Rekombinanter, replikationskompetenter Adenovirus-Vektor nach einem der Ansprüche 1 bis 5 zur Verwendung als ein Arzneimittel.

9. Rekombinanter, replikationskompetenter Adenovirus-Vektor nach einem der Ansprüche 1 bis 5 zur Verwendung bei der Behandlung einer Krankheit, die aus der Gruppe bestehend aus einer infektiösen Viruserkrankung, einer infektiösen bakteriellen Erkrankung, einer Krebserkrankung, einer Gefäßerkrankung, einer kardiovaskulären Erkrankung, einer immunologischen Erkrankung, einer Lebererkrankung und einer parasitären Erkrankung ausgewählt ist.

10. Rekombinanter, replikationskompetenter Adenovirus-Vektor zur Verwendung nach Anspruch 9, wobei die infektiöse Viruserkrankung aus der Gruppe bestehend aus Viruserkrankungen, die durch HCV, HBV, HIV und das Influenza-A-Virus verursacht werden, ausgewählt ist.

11. Rekombinanter, replikationskompetenter Adenovirus-Vektor zur Verwendung nach Anspruch 9, wobei die infektiöse bakterielle Erkrankung aus der Gruppe bestehend aus Tuberkulose und septischem Schock ausgewählt ist.

12. Rekombinanter, replikationskompetenter Adenovirus-Vektor zur Verwendung nach Anspruch 9, wobei die Krebserkrankung aus der Gruppe bestehend aus Lungen-krebs, Brustkrebs, Eierstockkrebs, Prostatakrebs, kolorektalem Krebs, Blasenkrebs, Nierenkrebs, hämatologischen Malignitäten, Leberkrebs, Bauchspeicheldrüsenkrebs, Tumoren neuralen Ursprungs, Kopf- und Halskrebs und Osteosarkom ausgewählt ist.

13. Rekombinanter, replikationskompetenter Adenovirus-Vektor nach einem der Ansprüche 1 bis 5 zur Verwendung in einer Gentherapie.

14. Säugetierzelle, umfassend einen rekombinanten, replikationskompetenten Adenovirus-Vektor nach einem der Ansprüche 1 bis 5.

15. Verfahren zum Exprimieren eines Proteins oder Genprodukts oder Expressionsprodukts, welches das Infizieren oder Transfizieren einer Zelle in vitro mit einem rekombinanten, replikationskompetenten Adenovirus-Vektor nach einem der Ansprüche 1 bis 5 und gegebenenfalls das Extrahieren, Reinigen oder Isolieren des Proteins, Genprodukts oder Expressionsprodukts aus der Zelle umfasst.

16. Verwendung eines rekombinanten, replikationskompetenten Adenovirus-Vektors nach einem der Ansprüche 1 bis 5 zur Invitro-Identifizierung einer antiviralen Verbindung.

## Revendications

1. Vecteur d'adénovirus Ad11p recombinant compétent pour la réplication, qui contient une séquence d'acide nucléique isolée possédant au moins 90 %, de manière préférée au moins 95 %, de manière davantage préférée au moins 98 % et de manière préférée entre toutes au moins 99 % d'identité de séquence à la séquence présentée dans SEQ ID n°1, et contenant en outre un ou plusieurs inserts d'un/de fragment(s) d'acide nucléique hétérologue(s) qui est/sont soit (i) cloné(s) entre le nucléotide 247 et le nucléotide 568 de la SEQ ID n°1 d'un vecteur de ce genre et dont le vecteur exprime l'intégralité du génome viral, soit (ii) dans lequel ledit fragment d'acide nucléique hétérologue remplace la séquence entre les nucléotides 28536 et 29482 de la SEQ ID n°1 dans ledit vecteur.

2. Vecteur d'adénovirus Ad11p recombinant compétent pour la réplication selon la revendication 1, dans lequel ledit insert possède une taille comprise entre 100 et 5 000 paires de bases, par exemple comprise entre 500 et 2 000 paires de bases.

3. Vecteur d'adénovirus Ad11p recombinant compétent pour la réplication selon l'une quelconque des revendications 1 à 2, dans lequel ledit insert encode un siARN, un shARN ou un micro ARN.

4. Vecteur d'adénovirus Ad11p recombinant compétent pour la réplication selon l'une quelconque des revendications 1 à 3, dans lequel ledit fragment d'acide nucléique hétérologue encode une protéine sélectionnée dans le groupe comprenant : une protéine virale, un déterminant antigénique d'un organisme pathogène, un antigène spécifique de tumeur, une protéine humaine, une cytokine.

5. Vecteur d'adénovirus Ad11p recombinant compétent pour la réplication selon l'une quelconque des revendications 1 à 4, dans lequel ledit fragment d'acide nucléique hétérologue encode une protéine rapporteuse sélectionnée dans le groupe comprenant : une protéine fluorescente, une protéine luminescente, et une enzyme.

6. Composition pharmaceutique comprenant un vecteur d'adénovirus recombinant compétent pour la réplication selon l'une quelconque des revendications 1 à 5, et un excipient approprié.

7. Composition pharmaceutique selon la revendication 6 formulée pour une administration locale sur un site tumoral, pour une administration intravésicale ou intracanalaire, ou injection intraveineuse.

8. Vecteur d'adénovirus recombinant compétent pour la réplication selon l'une quelconque des revendications 1 à 5 pour une utilisation en tant que médicament.

9. Vecteur d'adénovirus recombinant compétent pour la réplication selon l'une quelconque des revendications 1 à 5 pour une utilisation dans le traitement d'une maladie sélectionnée dans le groupe comprenant : une maladie infectieuse virale, une maladie infectieuse bactérienne, une maladie cancéreuse, une maladie vasculaire, une maladie cardiovasculaire, une maladie immunitaire, une maladie hépatique, et une maladie parasitaire.

10. Vecteur d'adénovirus recombinant compétent pour la réplication pour une utilisation selon la revendication 9, dans lequel ladite maladie infectieuse virale est sélectionnée dans le groupe comprenant des maladies virales causées par le virus de l'hépatite C, le virus de l'hépatite B, le VIH et le virus de la grippe A.

11. Vecteur d'adénovirus recombinant compétent pour la réplication pour une utilisation selon la revendication 9, dans lequel ladite maladie infectieuse bactérienne est sélectionnée dans le groupe comprenant la tuberculose et le choc septique.

12. Vecteur d'adénovirus recombinant compétent pour la réplication pour une utilisation selon la revendication 9, dans lequel ladite maladie cancéreuse est sélectionnée dans le groupe comprenant le cancer du poumon, le cancer du sein, le cancer des ovaires, le cancer de la prostate, le cancer colorectal, le cancer de la vessie, le cancer du rein, les hémopathies malignes, le cancer hépatique, le cancer du pancréas, les tumeurs d'origine neurologique, le cancer des voies aérodigestives supérieures, et l'ostéosarcome.

13. Vecteur d'adénovirus recombinant compétent pour la réplication selon l'une quelconque des revendications 1 à 5 pour une utilisation dans une thérapie génique.

14. Cellule de mammifère contenant un vecteur d'adénovirus recombinant compétent pour la réplication selon l'une quelconque des revendications 1 à 5.

15. Procédé d'expression d'un produit protéinique ou génique ou un produit d'expression qui consiste à infecter ou transfecter une cellule in vitro avec un vecteur d'adénovirus recombinant compétent pour la réplication selon l'une quelconque des revendications 1 à 5 et facultativement extraire, purifier ou isoler la protéine, le produit génique ou le produit d'expression à partir de la cellule.

16. Utilisation d'un vecteur d'adénovirus recombinant compétent pour la réplication selon l'une quelconque des revendications 1 à 5 pour l'identification in vitro d'un composé antiviral.


### SEQUENCE LISTING

<110> WADELL, GÖRAN MEI, YA-FANG
<120> REPLICATING VIRAL VECTORS FOR GENE THERAPY
<130> 223286-229192
<150> US 61/248,516
   <151> 2009-10-05
<160> 1
<170> PatentIn version 3.3
<210> 1
   <211> 34794
   <212> DNA
   <213> ADENOVIRUS 11p
<400> 1
   catcatcaat aatatacctt atagatggaa tggtgccaat atgtaaatga ggtgatttta      60
   aaaagtgtgg atcgtgtggt gattggctgt ggggttaacg gctaaaaggg gcggtgcgac     120
   cgtgggaaaa tgacgttttg tgggggtgga gtttttttgc aagttgtcgc gggaaatgtg     180
   acgcataaaa aggctttttt ctcacggaac tacttagttt tcccacggta tttaacagga     240
   aatgaggtag ttttgaccgg atgcaagtga aaattgttga ttttcgcgcg aaaactgaat     300
   gaggaagtgt ttttctgaat aatgtggtat ttatggcagg gtggagtatt tgttcagggc     360
   caggtagact ttgacccatt acgtggaggt ttcgattacc gtgtttttta cctgaatttc     420
   cgcgtaccgt gtcaaagtct tctgttttta cgtaggtgtc agctgatcgc tagggtattt     480
   atacctcagg gtttgtgtca agaggccact cttgagtgcc agcgagaaga gttttctcct     540
   ctgcgccggc agtttaataa taaaaaaatg agagatttgc gatttctgcc tcaggaaata     600
   atctctgctg agactggaaa tgaaatattg gagcttgtgg tgcacgccct gatgggagac     660
   gatccggagc cacctgtgca gctttttgag cctcctacgc ttcaggaact gtatgattta     720
   gaggtagagg gatcggagga ttctaatgag gaagctgtaa atggcttttt taccgattct     780
   atgcttttag ctgctaatga agggttagaa ttagatccgc ctttggacac ttttgatact     840
   ccaggggtaa ttgtggaaag cggtacaggt gtaagaaaat tacctgattt gagttccgtg     900
   gactgtgatt tgcactgcta tgaagacggg tttcctccga gtgatgagga ggaccatgaa     960
   aaggagcagt ccatgcagac tgcagcgggt gagggagtga aggctgccaa tgttggtttt    1020
   cagttggatt gcccggagct tcctggacat ggctgtaagt cttgtgaatt tcacaggaaa    1080
   aatactggag taaaggaact gttatgttcg ctttgttata tgagaacgca ctgccacttt    1140
   atttacagta agtgtgttta agttaaaatt taaaggaata tgctgttttt cacatgtata    1200
   ttgagtgtga gttttgtgct tcttattata ggtcctgtgt ctgatgctga tgaatcacca    1260
   tctcctgatt ctactacctc acctcctgag attcaagcac ctgttcctgt ggacgtgcgc    1320
   aagcccattc ctgtgaagct taagcctggg aaacgtccag cagtggaaaa acttgaggac 1380
   ttgttacagg gtggggacgg acctttggac ttgagtacac ggaaacgtcc aagacaataa 1440
   gtgttccata tccgtgttta cttaaggtga cgtcaatatt tgtgtgacag tgcaatgtaa 1500
   taaaaatatg ttaactgttc actggttttt attgcttttt gggcggggac tcaggtatat 1560
   aagtagaagc agacctgtgt ggttagctca taggagctgg ctttcatcca tggaggtttg 1620
   ggccattttg gaagacctta ggaagactag gcaactgtta gagaacgctt cggacggagt 1680
   ctccggtttt tggagattct ggttcgctag tgaattagct agggtagttt ttaggataaa 1740
   acaggactat aaacaagaat ttgaaaagtt gttggtagat tgcccaggac tttttgaagc 1800
   tcttaatttg ggccatcagg ttcactttaa agaaaaagtt ttatcagttt tagacttttc 1860
   aaccccaggt agaactgctg ctgctgtggc ttttcttact tttatattag ataaatggat 1920
   cccgcagact catttcagca ggggatacgt tttggatttc atagccacag cattgtggag 1980
   aacatggaag gttcgcaaga tgaggacaat cttaggttac tggccagtgc agcctttggg 2040
   tgtagcggga atcctgaggc atccaccggt catgccagcg gttctggagg aggaacagca 2100
   agaggacaac ccgagagccg gcctggaccc tccagtggag gaggcggagt agctgacttg 2160
   tctcctgaac tgcaacgggt gcttactgga tctacgtcca ctggacggga taggggcgtt 2220
   aagagggaga gggcatctag tggtactgat gctagatctg agttggcttt aagtttaatg 2280
   agtcgcagac gtcctgaaac catttggtgg catgaggttc agaaagaggg aagggatgaa 2340
   gtttctgtat tgcaggagaa atattcactg gaacaggtga aaacatgttg gttggagcct 2400
   gaggatgatt gggaggtggc cattaaaaat tatgccaaga tagctttgag gcctgataaa 2460
   cagtataaga ttactagacg gattaatatc cggaatgctt gttacatatc tggaaatggg 2520
   gctgaggtgg taatagatac tcaagacaag gcagttatta gatgctgcat gatggatatg 2580
   tggcctgggg tagtcggtat ggaagcagta acttttgtaa atgttaagtt taggggagat 2640
   ggttataatg gaatagtgtt tatggccaat accaaactta tattgcatgg ttgtagcttt 2700
   tttggtttca acaatacctg tgtagatgcc tggggacagg ttagtgtacg gggatgtagt 2760
   ttctatgcgt gttggattgc cacagctggc agaaccaaga gtcaattgtc tctgaagaaa 2820
   tgcatatttc aaagatgtaa cctgggcatt ctgaatgaag gcgaagcaag ggtccgccac 2880
   tgcgcttcta cagatactgg atgttttatt ttgattaagg gaaatgccag cgtaaagcat 2940
   aacatgattt gcggtgcttc cgatgagagg ccttatcaaa tgctcacttg tgctggtggg 3000
   cattgtaata tgctggctac tgtgcatatt gtttcccatc aacgcaaaaa atggcctgtt 3060
   tttgatcaca atgtgatgac gaagtgtacc atgcatgcag gtgggcgtag aggaatgttt 3120
   atgccttacc agtgtaacat gaatcatgtg aaagtgttgt tggaaccaga tgccttttcc 3180
   agaatgagcc taacaggaat ttttgacatg aacatgcaaa tctggaagat cctgaggtat 3240
   gatgatacga gatcgagggt acgcgcatgc gaatgcggag gcaagcatgc caggttccag 3300
   ccggtgtgtg tagatgtgac tgaagatctc agaccggatc atttggttat tgcccgcact 3360
   ggagcagagt tcggatccag tggagaagaa actgactaag gtgagtattg ggaaaacttt 3420
   ggggtgggat tttcagatgg acagattgag taaaaatttg ttttttctgt cttgcagctg 3480
   tcatgagtgg aaacgcttct tttaaggggg gagtcttcag cccttatctg acagggcgtc 3540
   tcccatcctg ggcaggagtt cgtcagaatg ttatgggatc tactgtggat ggaagacccg 3600
   tccaacccgc caattcttca acgctgacct atgctacttt aagttcttca cctttggacg 3660
   cagctgcagc tgccgccgcc gcttctgttg ccgctaacac tgtgcttgga atgggttact 3720
   atggaagcat catggctaat tccacttcct ctaataaccc ttctaccctg actcaggaca 3780
   agttacttgt ccttttggcc cagctggagg ctttgaccca acgtctgggt gaactttctc 3840
   agcaggtggt cgagttgcga gtacaaactg agtctgctgt cggcacggca aagtctaaat 3900
   aaaaaaatcc cagaatcaat gaataaataa acaagcttgt tgttgattta aaatcaagtg 3960
   tttttatttc atttttcgcg cacggtatgc cctagaccac cgatctctat cattgagaac 4020
   tcggtggatt ttttccagga tcctatagag gtgggattga atgtttagat acatgggcat 4080
   taggccgtct ttggggtgga gatagctcca ttgaagggat tcatgctccg gggtagtgtt 4140
   gtaaatcacc cagtcataac aaggtcgcag tgcatggtgt tgcacaatat cttttagaag 4200
   taggctgatt gccacagata agcccttggt gtaggtgttt acaaaccggt tgagctggga 4260
   tgggtgcatt cggggtgaaa ttatgtgcat tttggattgg atttttaagt tggcaatatt 4320
   gccgccaaga tcccgtcttg ggttcatgtt atgaaggacc accaagacgg tgtatccggt 4380
   acatttagga aatttatcgt gcagcttgga tggaaaagcg tggaaaaatt tggagacacc 4440
   cttgtgtcct ccaagatttt ccatgcactc atccatgata atagcaatgg ggccgtgggc 4500
   agcggcgcgg gcaaacacgt tccgtgggtc tgacacatca tagttatgtt cctgagttaa 4560
   atcatcataa gccattttaa tgaatttggg gcggagagta ccagattggg gtatgaatgt 4620
   tccttcgggc cccggagcat agttcccctc acagatttgc atttcccaag ctttcagttc 4680
   cgagggtgga atcatgtcca cctggggggc tatgaaaaac accgtttctg gggcgggggt 4740
   gattaattgt gatgatagca aatttctgag caattgagat ttgccacatc cggtggggcc 4800
   ataaatgatt ccgattacgg gttgcaggtg gtagtttagg gaacggcaac tgccgtcttc 4860
   tcgaagcaag ggggccacct cgttcatcat ttcccttaca tgcatatttt cccgcaccaa 4920
   atccattagg aggcgctctc ctcctagtga tagaagttct tgtagtgagg aaaagttttt 4980
   cagcggtttc agaccgtcag ccatgggcat tttggagaga gtttgctgca aaagttctag 5040
   tctgttccac agttcagtga tgtgttctat ggcatctcga tccagcagac ctcctcgttt 5100
   cgcgggtttg gacggctcct ggaatagggt atgagacgat gggcgtccag cgctgccagg 5160
   gttcggtcct tccagggtct cagtgttcga gtcagggttg tttccgtcac agtgaagggg 5220
   tgtgcgcctg cttgggcgct tgccagggtg cgcttcagac tcatcctgct ggtcgaaaac 5280
   ttctgtcgct tggcgccctg tatgtcggcc aagtagcagt ttaccatgag ttcgtagttg 5340
   agcgcctcgg ctgcgtggcc tttggcgcgg agcttacctt tggaagtttt cttgcatacc 5400
   gggcagtata ggcatttcag cgcatacaac ttgggcgcaa ggaaaacgga ttctggggag 5460
   tatgcatctg cgccgcagga ggcgcaaaca gtttcacatt ccaccagcca ggttaaatcc 5520
   ggttcattgg ggtcaaaaac aagttttccg ccatattttt tgatgcgttt cttacctttg 5580
   gtctccatga gttcgtgtcc tcgttgagtg acaaacaggc tgtccgtgtc cccgtagact 5640
   gattttacag gcctcttctc cagtggagtg cctcggtctt cttcgtacag gaactctgac 5700
   cactctgata caaaggcgcg cgtccaggcc agcacaaagg aggctatgtg ggaggggtag 5760
   cgatcgttgt caaccagggg gtccaccttt tccaaagtat gcaaacacat gtcaccctct 5820
   tcaacatcca ggaatgtgat tggcttgtag gtgtatttca cgtgacctgg ggtccccgct 5880
   gggggggtat aaaagggggc ggttctttgc tcttcctcac tgtcttccgg atcgctgtcc 5940
   aggaacgtca gctgttgggg taggtattcc ctctcgaagg cgggcatgac ctctgcactc 6000
   aggttgtcag tttctaagaa cgaggaggat ttgatattga cagtgccggt tgagatgcct 6060
   ttcatgaggt tttcgtccat ttggtcagaa aacacaattt ttttattgtc aagtttggtg 6120
   gcaaatgatc catacagggc gttggataaa agtttggcaa tggatcgcat ggtttggttc 6180
   ttttccttgt ccgcgcgctc tttggcggcg atgttgagtt ggacatactc gcgtgccagg 6240
   cacttccatt cggggaagat agttgttaat tcatctggca cgattctcac ttgccaccct 6300
   cgattatgca aggtaattaa atccacactg gtggccacct cgcctcgaag gggttcattg 6360
   gtccaacaga gcctacctcc tttcctagaa cagaaagggg gaagtgggtc tagcataagt 6420
   tcatcgggag ggtctgcatc catggtaaag attcccggaa gtaaatcctt atcaaaatag 6480
   ctgatgggag tggggtcatc taaggccatt tgccattctc gagctgccag tgcgcgctca 6540
   tatgggttaa ggggactgcc ccatggcatg ggatgggtga gtgcagaggc atacatgcca 6600
   cagatgtcat agacgtagat gggatcctca aagatgccta tgtaggttgg atagcatcgc 6660
   ccccctctga tacttgctcg cacatagtca tatagttcat gtgatggcgc tagcagcccc 6720
   ggacccaagt tggtgcgatt gggtttttct gttctgtaga cgatctggcg aaagatggcg 6780
   tgagaattgg aagagatggt gggtctttga aaaatgttga aatgggcatg aggtagacct 6840
   acagagtctc tgacaaagtg ggcataagat tcttgaagct tggttaccag ttcggcggtg 6900
   acaagtacgt ctagggcgca gtagtcaagt gtttcttgaa tgatgtcata acctggttgg 6960
   tttttctttt cccacagttc gcggttgaga aggtattctt cgcgatcctt ccagtactct 7020
   tctagcggaa acccgtcttt gtctgcacgg taagatccta gcatgtagaa ctgattaact 7080
   gccttgtaag ggcagcagcc cttctctacg ggtagagagt atgcttgagc agcttttcgt 7140
   agcgaagcgt gagtaagggc aaaggtgtct ctgaccatga ctttgagaaa ttggtatttg 7200
   aagtcgatgt cgtcacaggc tccctgttcc cagagttgga agtctacccg tttcttgtag 7260
   gcggggttgg gcaaagcgaa agtaacatca ttgaagagaa tcttaccggc tctgggcata 7320
   aaattgcgag tgatgcgaaa aggctgtggt acttccgctc gattgttgat cacctgggca 7380
   gctaggacga tctcgtcgaa accgttgatg ttgtgtccta cgatgtataa ttctatgaaa 7440
   cgcggcgtgc ctctgacgtg aggtagctta ctgagctcat caaaggttag gtctgtgggg 7500
   tcagataagg cgtagtgttc gagagcccat tcgtgcaggt gaggatttgc atgtaggaat 7560
   gatgaccaaa gatctaccgc cagtgctgtt tgtaactggt cccgatactg acgaaaatgc 7620
   cggccaattg ccattttttc tggagtgaca cagtagaagg ttctggggtc ttgttgccat 7680
   cgatcccact tgagtttaat ggctagatcg tgggccatgt tgacgagacg ctcttctcct 7740
   gagagtttca tgaccagcat gaaaggaact agttgtttgc caaaggatcc catccaggtg 7800
   taagtttcca catcgtaggt caggaagagt ctttctgtgc gaggatgaga gccgatcggg 7860
   aagaactgga tttcctgcca ccagttggag gattggctgt tgatgtgatg gaagtagaag 7920
   tttctgcggc gcgccgagca ttcgtgtttg tgcttgtaca gacggccgca gtagtcgcag 7980
   cgttgcacgg gttgtatctc gtgaatgagt tgtacctggc ttcccttgac gagaaatttc 8040
   agtgggaagc cgaggcctgg cgattgtatc tcgtgctctt ctatattcgc tgtatcggcc 8100
   tgttcatctt ctgtttcgat ggtggtcatg ctgacgagcc cccgcgggag gcaagtccag 8160
   acctcggcgc gggaggggcg gagctgaagg acgagagcgc gcaggctgga gctgtccaga 8220
   gtcctgagac gctgcggact caggttagta ggtagggaca gaagattaac ttgcatgatc 8280
   ttttccaggg cgtgcgggag gttcagatgg tacttgattt ccacaggttc gtttgtagag 8340
   acgtcaatgg cttgcagggt tccgtgtcct ttgggcgcca ctaccgtacc tttgtttttt 8400
   cttttgatcg gtggtggctc tcttgcttct tgcatgctca gaagcggtga cggggacgcg 8460
   cgccgggcgg cagcggttgt tccggacccg agggcatggc tggtagtggc acgtcggcgc 8520
   cgcgcacggg caggttctgg tactgcgctc tgagaagact tgcgtgcgcc accacgcgtc 8580
   gattgacgtc ttgtatctga cgtctctggg tgaaagctac cggccccgtg agcttgaacc 8640
   tgaaagagag ttcaacagaa tcaatttcgg tatcgttaac ggcagcttgt ctcagtattt 8700
   cttgtacgtc accagagttg tcctggtagg cgatctccgc catgaactgc tcgatttctt 8760
   cctcctgaag atctccgcga cccgctcttt cgacggtggc cgcgaggtca ttggagatac 8820
   ggcccatgag ttgggagaat gcattcatgc ccgcctcgtt ccagacgcgg ctgtaaacca 8880
   cggccccctc ggagtctctt gcgcgcatca ccacctgagc gaggttaagc tccacgtgtc 8940
   tggtgaagac cgcatagttg cataggcgct gaaaaaggta gttgagtgtg gtggcaatgt 9000
   gttcggcgac gaagaaatac atgatccatc gtctcagcgg catttcgcta acatcgccca 9060
   gagcttccaa gcgctccatg gcctcgtaga agtccacggc aaaattaaaa aactgggagt 9120
   ttcgcgcgga cacggtcaat tcctcctcga gaagacggat gagttcggct atggtggccc 9180
   gtacttcgcg ttcgaaggct cccgggatct cttcttcctc ttctatctct tcttccacta 9240
   acatctcttc ttcgtcttca ggcgggggcg gagggggcac gcggcgacgt cgacggcgca 9300
   cgggcaaacg gtcgatgaat cgttcaatga cctctccgcg gcggcggcgc atggtttcag 9360
   tgacggcgcg gccgttctcg cgcggtcgca gagtaaaaac accgccgcgc atctccttaa 9420
   agtggtgact gggaggttct ccgtttggga gggagagggc gctgattata cattttatta 9480
   attggcccgt agggactgca cgcagagatc tgatcgtgtc aagatccacg ggatctgaaa 9540
   acctttcgac gaaagcgtct aaccagtcac agtcacaagg taggctgagt acggcttctt 9600
   gtgggcgggg gtggttatgt gttcggtctg ggtcttctgt ttcttcttca tctcgggaag 9660
   gtgagacgat gctgctggtg atgaaattaa agtaggcagt tctaagacgg cggatggtgg 9720
   cgaggagcac caggtctttg ggtccggctt gctggatacg caggcgattg gccattcccc 9780
   aagcattatc ctgacatcta gcaagatctt tgtagtagtc ttgcatgagc cgttctacgg 9840
   gcacttcttc ctcacccgtt ctgccatgca tacgtgtgag tccaaatccg cgcattggtt 9900
   gtaccagtgc caagtcagct acgactcttt cggcgaggat ggcttgctgt acttgggtaa 9960
   gggtggcttg aaagtcatca aaatccacaa agcggtggta agctcctgta ttaatggtgt 10020
   aagcacagtt ggccatgact gaccagttaa ctgtctggtg accagggcgc acgagctcgg 10080
   tgtatttaag gcgcgaatag gcgcgggtgt caaagatgta atcgttgcag gtgcgcacca 10140
   gatactggta ccctataaga aaatgcggcg gtggttggcg gtagagaggc catcgttctg 10200
   tagctggagc gccaggggcg aggtcttcca acataaggcg gtgatagccg tagatgtacc 10260
   tggacatcca ggtgattcct gcggcggtag tagaagcccg aggaaactcg cgtacgcggt 10320
   tccaaatgtt gcgtagcggc atgaagtagt tcattgtagg cacggtttga ccagtgaggc 10380
   gcgcgcagtc attgatgctc tatagacacg gagaaaatga aagcgttcag cgactcgact 10440
   ccgtagcctg gaggaacgtg aacgggttgg gtcgcggtgt accccggttc gagacttgta 10500
   ctcgagccgg ccggagccgc ggctaacgtg gtattggcac tcccgtctcg acccagccta 10560
   caaaaatcca ggatacggaa tcgagtcgtt ttgctggttt ccgaatggca gggaagtgag 10620
   tcctattttt ttttttttgc cgctcagatg catcccgtgc tgcgacagat gcgcccccaa 10680
   caacagcccc cctcgcagca gcagcagcag caatcacaaa aggctgtccc tgcaactact 10740
   gcaactgccg ccgtgagcgg tgcgggacag cccgcctatg atctggactt ggaagagggc 10800
   gaaggactgg cacgtctagg tgcgccttca cccgagcggc atccgcgagt tcaactgaaa 10860
   aaagattctc gcgaggcgta tgtgccccaa cagaacctat ttagagacag aagcggcgag 10920
   gagccggagg agatgcgagc ttcccgcttt aacgcgggtc gtgagctgcg tcacggtttg 10980
   gaccgaagac gagtgttgcg ggacgaggat ttcgaagttg atgaaatgac agggatcagt 11040
   cctgccaggg cacacgtggc tgcagccaac cttgtatcgg cttacgagca gacagtaaag 11100
   gaagagcgta acttccaaaa gtcttttaat aatcatgtgc gaaccctgat tgcccgcgaa 11160
   gaagttaccc ttggtttgat gcatttgtgg gatttgatgg aagctatcat tcagaaccct 11220
   actagcaaac ctctgaccgc ccagctgttt ctggtggtgc aacacagcag agacaatgag 11280
   gctttcagag aggcgctgct gaacatcacc gaacccgagg ggagatggtt gtatgatctt 11340
   atcaacattc tacagagtat catagtgcag gagcggagcc tgggcctggc cgagaaggtg 11400
   gctgccatca attactcggt tttgagcttg ggaaaatatt acgctcgcaa aatctacaag 11460
   actccatacg ttcccataga caaggaggtg aagatagatg ggttctacat gcgcatgacg 11520
   ctcaaggtct tgaccctgag cgatgatctt ggggtgtatc gcaatgacag aatgcatcgc 11580
   gcggttagcg ccagcaggag gcgcgagtta agcgacaggg aactgatgca cagtttgcaa 11640
   agagctctga ctggagctgg aaccgagggt gagaattact tcgacatggg agctgacttg 11700
   cagtggcagc ctagtcgcag ggctctgagc gccgcgacgg caggatgtga gcttccttac 11760
   atagaagagg cggatgaagg cgaggaggaa gagggcgagt acttggaaga ctgatggcac 11820
   aacccgtgtt ttttgctaga tggaacagca agcaccggat cccgcaatgc gggcggcgct 11880
   gcagagccag ccgtccggca ttaactcctc ggacgattgg acccaggcca tgcaacgtat 11940
   catggcgttg acgactcgca accccgaagc ctttagacag caaccccagg ccaaccgtct 12000
   atcggccatc atggaagctg tagtgccttc ccgctctaat cccactcatg agaaggtcct 12060
   ggccatcgtg aacgcgttgg tggagaacaa agctattcgt ccagatgagg ccggactggt 12120
   atacaacgct ctcttagaac gcgtggctcg ctacaacagt agcaatgtgc aaaccaattt 12180
   ggaccgtatg ataacagatg tacgcgaagc cgtgtctcag cgcgaaaggt tccagcgtga 12240
   tgccaacctg ggttcgctgg tggcgttaaa tgctttcttg agtactcagc ctgctaatgt 12300
   gccgcgtggt caacaggatt atactaactt tttaagtgct ttgagactga tggtatcaga 12360
   agtacctcag agcgaagtgt atcagtccgg tcctgattac ttctttcaga ctagcagaca 12420
   gggcttgcag acggtaaatc tgagccaagc ttttaaaaac cttaaaggtt tgtggggagt 12480
   gcatgccccg gtaggagaaa gagcaaccgt gtctagcttg ttaactccga actcccgcct 12540
   attattactg ttggtagctc ctttcaccga cagcggtagc atcgaccgta attcctattt 12600
   gggttaccta ctaaacctgt atcgcgaagc catagggcaa agtcaggtgg acgagcagac 12660
   ctatcaagaa attacccaag tcagtcgcgc tttgggacag gaagacactg gcagtttgga 12720
   agccactctg aacttcttgc ttaccaatcg gtctcaaaag atccctcctc aatatgctct 12780
   tactgcggag gaggagagga tccttagata tgtgcagcag agcgtgggat tgtttctgat 12840
   gcaagagggg gcaactccga ctgcagcact ggacatgaca gcgcgaaata tggagcccag 12900
   catgtatgcc agtaaccgac ctttcattaa caaactgctg gactacttgc acagagctgc 12960
   cgctatgaac tctgattatt tcaccaatgc catcttaaac ccgcactggc tgcccccacc 13020
   tggtttctac acgggcgaat atgacatgcc cgaccctaat gacggatttc tgtgggacga 13080
   cgtggacagc gatgtttttt cacctctttc tgatcatcgc acgtggaaaa aggaaggcgg 13140
   cgatagaatg cattcttctg catcgctgtc cggggtcatg ggtgctaccg cggctgagcc 13200
   cgagtctgca agtccttttc ctagtctacc cttttctcta cacagtgtac gtagcagcga 13260
   agtgggtaga ataagtcgcc cgagtttaat gggcgaagag gagtatctaa acgattcctt 13320
   gctcagaccg gcaagagaaa aaaatttccc aaacaatgga atagaaagtt tggtggataa 13380
   aatgagtaga tggaagactt atgctcagga tcacagagac gagcctggga tcatggggat 13440
   tacaagtaga gcgagccgta gacgccagcg ccatgacaga cagaggggtc ttgtgtggga 13500
   cgatgaggat tcggccgatg atagcagcgt gctggacttg ggtgggagag gaaggggcaa 13560
   cccgtttgct catttgcgcc ctcgcttggg tggtatgttg taaaaaaaaa taaaaaaaaa 13620
   actcaccaag gccatggcga cgagcgtacg ttcgttcttc tttattatct gtgtctagta 13680
   taatgaggcg agtcgtgcta ggcggagcgg tggtgtatcc ggagggtcct cctccttcgt 13740
   acgagagcgt gatgcagcag cagcaggcga cggcggtgat gcaatcccca ctggaggctc 13800
   cctttgtgcc tccgcgatac ctggcaccta cggagggcag aaacagcatt cgttattcgg 13860
   aactggcacc tcagtacgat accaccaggt tgtatctggt ggacaacaag tcggcggaca 13920
   ttgcttctct gaactatcag aatgaccaca gcaacttctt gaccacggtg gtgcaaaaca 13980
   atgactttac ccctacggaa gccagcaccc agaccattaa ctttgatgaa cgatcgcggt 14040
   ggggcggtca gctaaagacc atcatgcata ctaacatgcc aaacgtgaac gagtatatgt 14100
   ttagtaacaa gttcaaagcg cgtgtgatgg tgtccagaaa acctcccgac ggtgctgcag 14160
   ttggggatac ttatgatcac aagcaggata ttttgaaata tgagtggttc gagtttactt 14220
   tgccagaagg caacttttca gttactatga ctattgattt gatgaacaat gccatcatag 14280
   ataattactt gaaagtgggt agacagaatg gagtgcttga aagtgacatt ggtgttaagt 14340
   tcgacaccag gaacttcaag ctgggatggg atcccgaaac caagttgatc atgcctggag 14400
   tgtatacgta tgaagccttc catcctgaca ttgtcttact gcctggctgc ggagtggatt 14460
   ttaccgagag tcgtttgagc aaccttcttg gtatcagaaa aaaacagcca tttcaagagg 14520
   gttttaagat tttgtatgaa gatttagaag gtggtaatat tccggccctc ttggatgtag 14580
   atgcctatga gaacagtaag aaagaacaaa aagccaaaat agaagctgct acagctgctg 14640
   cagaagctaa ggcaaacata gttgccagcg actctacaag ggttgctaac gctggagagg 14700
   tcagaggaga caattttgcg ccaacacctg ttccgactgc agaatcatta ttggccgatg 14760
   tgtctgaagg aacggacgtg aaactcacta ttcaacctgt agaaaaagat agtaagaata 14820
   gaagctataa tgtgttggaa gacaaaatca acacagccta tcgcagttgg tatctttcgt 14880
   acaattatgg cgatcccgaa aaaggagtgc gttcctggac attgctcacc acctcagatg 14940
   tcacctgcgg agcagagcag gtctactggt cgcttccaga catgatgaag gatcctgtca 15000
   ctttccgctc cactagacaa gtcagtaact accctgtggt gggtgcagag cttatgcccg 15060
   tcttctcaaa gagcttctac aacgaacaag ctgtgtactc ccagcagctc cgccagtcca 15120
   cctcgcttac gcacgtcttc aaccgctttc ctgagaacca gattttaatc cgtccgccgg 15180
   cgcccaccat taccaccgtc agtgaaaacg ttcctgctct cacagatcac gggaccctgc 15240
   cgttgcgcag cagtatccgg ggagtccaac gtgtgaccgt tactgacgcc agacgccgca 15300
   cctgtcccta cgtgtacaag gcactgggca tagtcgcacc gcgcgtcctt tcaagccgca 15360
   ctttctaaaa aaaaaaaaaa tgtccattct tatctcgccc agtaataaca ccggttgggg 15420
   tctgcgcgct ccaagcaaga tgtacggagg cgcacgcaaa cgttctaccc aacatcctgt 15480
   ccgtgttcgc ggacattttc gcgctccatg gggcgccctc aagggccgca ctcgcgttcg 15540
   aaccaccgtc gatgatgtaa tcgatcaggt ggttgccgac gcccgtaatt atactcctac 15600
   tgcgcctaca tctactgtgg atgcagttat tgacagtgta gtggctgacg ctcgcaacta 15660
   tgctcgacgt aagagccggc gaaggcgcat tgccagacgc caccgagcta ccactgccat 15720
   gcgagccgca agagctctgc tacgaagagc tagacgcgtg gggcgaagag ccatgcttag 15780
   ggcggccaga cgtgcagctt cgggcgccag cgccggcagg tcccgcaggc aagcagccgc 15840
   tttcgcagcg gcgactattg ccgacatggc ccaatcgcga agaggcaatg tatactgggt 15900
   gcgtgacgct gccaccggtc aacgtgtacc cgtgcgcacc cgtccccctc gcacttagaa 15960
   gatactgagc agtctccgat gttgtgtccc agcggcgagg atgtccaagc gcaaatacaa 16020
   ggaagaaatg ctgcaggtta tcgcacctga agtctacggc caaccgttga aggatgaaaa 16080
   aaaaccccgc aaaatcaagc gggttaaaaa ggacaaaaaa gaagaggaag atggcgatga 16140
   tgggctggcg gagtttgtgc gcgagtttgc cccacggcga cgcgtgcaat ggcgtgggcg 16200
   caaagttcga catgtgttga gacctggaac ttcggtggtc tttacacccg gcgagcgttc 16260
   aagcgctact tttaagcgtt cctatgatga ggtgtacggg gatgatgata ttcttgagca 16320
   ggcggctgac cgattaggcg agtttgctta tggcaagcgt agtagaataa cttccaagga 16380
   tgagacagtg tcgataccct tggatcatgg aaatcccacc cctagtctta aaccggtcac 16440
   tttgcagcaa gtgttacccg taactccgcg aacaggtgtt aaacgcgaag gtgaagattt 16500
   gtatcccact atgcaactga tggtacccaa acgccagaag ttggaggacg ttttggagaa 16560
   agtaaaagtg gatccagata ttcaacctga ggttaaagtg agacccatta agcaggtagc 16620
   gcctggtctg ggggtacaaa ctgtagacat taagattccc actgaaagta tggaagtgca 16680
   aactgaaccc gcaaagccta ctgccacctc cactgaagtg caaacggatc catggatgcc 16740
   catgcctatt acaactgacg ccgccggtcc cactcgaaga tcccgacgaa agtacggtcc 16800
   agcaagtctg ttgatgccca attatgttgt acacccatct attattccta ctcctggtta 16860
   ccgaggcact cgctactatc gcagccgaaa cagtacctcc cgccgtcgcc gcaagacacc 16920
   tgcaaatcgc agtcgtcgcc gtagacgcac aagcaaaccg actcccggcg ccctggtgcg 16980
   gcaagtgtac cgcaatggta gtgcggaacc tttgacactg ccgcgtgcgc gttaccatcc 17040
   gagtatcatc acttaatcaa tgttgccgct gcctccttgc agatatggcc ctcacttgtc 17100
   gccttcgcgt tcccatcact ggttaccgag gaagaaactc gcgccgtaga agagggatgt 17160
   tgggacgcgg aatgcgacgc tacaggcgac ggcgtgctat ccgcaagcaa ttgcggggtg 17220
   gttttttacc agccttaatt ccaattatcg ctgctgcaat tggcgcgata ccaggcatag 17280
   cttccgtggc ggttcaggcc tcgcaacgac attgacattg gaaaaaaacg tataaataaa 17340
   aaaaaaaaaa tacaatggac tctgacactc ctggtcctgt gactatgttt tcttagagat 17400
   ggaagacatc aatttttcat ccttggctcc gcgacacggc acgaagccgt acatgggcac 17460
   ctggagcgac atcggcacga gccaactgaa cgggggcgcc ttcaattgga gcagtatctg 17520
   gagcgggctt aaaaattttg gctcaaccat aaaaacatac gggaacaaag cttggaacag 17580
   cagtacagga caggcgctta gaaataaact taaagaccag aacttccaac aaaaagtagt 17640
   cgatgggata gcttccggca tcaatggagt ggtagatttg gctaaccagg ctgtgcagaa 17700
   aaagataaac agtcgtttgg acccgccgcc agcaacccca ggtgaaatgc aagtggagga 17760
   agaaattcct ccgccagaaa aacgaggcga caagcgtccg cgtcccgatt tggaagagac 17820
   gctggtgacg cgcgtagatg aaccgccttc ttatgaggaa gcaacgaagc ttggaatgcc 17880
   caccactaga ccgatagccc caatggccac cggggtgatg aaaccttctc agttgcatcg 17940
   acccgtcacc ttggatttgc cccctccccc tgctgctact gctgtacccg cttctaagcc 18000
   tgtcgctgcc ccgaaaccag tcgccgtagc caggtcacgt cccgggggcg ctcctcgtcc 18060
   aaatgcgcac tggcaaaata ctctgaacag catcgtgggt ctaggcgtgc aaagtgtaaa 18120
   acgccgtcgc tgcttttaat taaatatgga gtagcgctta acttgcctat ctgtgtatat 18180
   gtgtcattac acgccgtcac agcagcagag gaaaaaagga agaggtcgtg cgtcgacgct 18240
   gagttacttt caagatggcc accccatcga tgctgcccca atgggcatac atgcacatcg 18300
   ccggacagga tgcttcggag tacctgagtc cgggtctggt gcagttcgcc cgcgccacag 18360
   acacctactt caatctggga aataagttta gaaatcccac cgtagcgccg acccacgatg 18420
   tgaccaccga ccgtagccag cggctcatgt tgcgcttcgt gcccgttgac cgggaggaca 18480
   atacatactc ttacaaagtg cggtacaccc tggccgtggg cgacaacaga gtgctggata 18540
   tggccagcac gttctttgac attaggggtg tgttggacag aggtcccagt ttcaaaccct 18600
   attctggtac ggcttacaac tccctggctc ctaaaggcgc tccaaataca tctcagtgga 18660
   ttgcagaagg tgtaaaaaat acaactggtg aggaacacgt aacagaagag gaaaccaata 18720
   ctactactta cacttttggc aatgctcctg taaaagctga agctgaaatt acaaaagaag 18780
   gactcccagt aggtttggaa gtttcagatg aagaaagtaa accgatttat gctgataaaa 18840
   catatcagcc agaacctcag ctgggagatg aaacttggac tgaccttgat ggaaaaaccg 18900
   aaaagtatgg aggcagggct ctcaaacccg atactaagat gaaaccatgc tacgggtcct 18960
   ttgccaaacc tactaatgtg aaaggcggtc aggcaaaaca aaaaacaacg gagcagccaa 19020
   atcagaaagt cgaatatgat atcgacatgg agttttttga tgcggcatcg cagaaaacaa 19080
   acttaagtcc taaaattgtc atgtatgcag aaaatgtaaa tttggaaact ccagacactc 19140
   atgtagtgta caaacctgga acagaagaca caagttccga agctaatttg ggacaacaat 19200
   ctatgcccaa cagacccaac tacattggct tcagagataa ctttattgga cttatgtact 19260
   ataacagtac tggtaacatg ggggtgctgg ctggtcaagc gtctcagtta aatgcagtgg 19320
   ttgacttgca ggacagaaac acagaacttt cttaccaact cttgcttgac tctctgggcg 19380
   acagaaccag atactttagc atgtggaatc aggctgtgga cagttatgat cctgatgtac 19440
   gtgttattga aaatcatggt gtggaagatg aacttcccaa ctactgtttt ccactggacg 19500
   gcataggtgt tccaacaacc agttacaaat caatagttcc aaatggagac aatgcgccta 19560
   attggaagga acctgaagta aatggaacaa gtgagatcgg acagggtaat ttgtttgcca 19620
   tggaaattaa ccttcaagcc aatctatggc gaagtttcct ttattccaat gtggctctat 19680
   atctcccaga ctcgtacaaa tacaccccgt ccaatgtcac tcttccagaa aacaaaaaca 19740
   cctacgacta catgaacggg cgggtggtgc cgccatctct agtagacacc tatgtgaaca 19800
   ttggtgccag gtggtctctg gatgccatgg acaatgtcaa cccattcaac caccaccgta 19860
   acgctggctt gcgttaccga tccatgcttc tgggtaacgg acgttatgtg cctttccaca 19920
   tacaagtgcc tcaaaaattc ttcgctgtta aaaacctgct gcttctccca ggctcctaca 19980
   cttatgagtg gaactttagg aaggatgtga acatggttct acagagttcc ctcggtaacg 20040
   acctgcgggt agatggcgcc agcatcagtt tcacgagcat caacctctat gctacttttt 20100
   tccccatggc tcacaacacc gcttccaccc ttgaagccat gctgcggaat gacaccaatg 20160
   atcagtcatt caacgactac ctatctgcag ctaacatgct ctaccccatt cctgccaatg 20220
   caaccaatat tcccatttcc attccttctc gcaactgggc ggctttcaga ggctggtcat 20280
   ttaccagact gaaaaccaaa gaaactccct ctttggggtc tggatttgac ccctactttg 20340
   tctattctgg ttctattccc tacctggatg gtaccttcta cctgaaccac acttttaaga 20400
   aggtttccat catgtttgac tcttcagtga gctggcctgg aaatgacagg ttactatctc 20460
   ctaacgaatt tgaaataaag cgcactgtgg atggcgaagg ctacaacgta gcccaatgca 20520
   acatgaccaa agactggttc ttggtacaga tgctcgccaa ctacaacatc ggctatcagg 20580
   gcttctacat tccagaagga tacaaagatc gcatgtattc atttttcaga aacttccagc 20640
   ccatgagcag gcaggtggtt gatgaggtca attacaaaga cttcaaggcc gtcgccatac 20700
   cctaccaaca caacaactct ggctttgtgg gttacatggc tccgaccatg cgccaaggtc 20760
   aaccctatcc cgctaactat ccctatccac tcattggaac aactgccgta aatagtgtta 20820
   cgcagaaaaa gttcttgtgt gacagaacca tgtggcgcat accgttctcg agcaacttca 20880
   tgtctatggg ggcccttaca gacttgggac agaatatgct ctatgccaac tcagctcatg 20940
   ctctggacat gacctttgag gtggatccca tggatgagcc caccctgctt tatcttctct 21000
   tcgaagtttt cgacgtggtc agagtgcatc agccacaccg cggcatcatc gaggcagtct 21060
   acctgcgtac accgttctcg gccggtaacg ctaccacgta agaagcttct tgcttcttgc 21120
   aaatagcagc tgcaaccatg gcctgcggat cccaaaacgg ctccagcgag caagagctca 21180
   gagccattgt ccaagacctg ggttgcggac cctatttttt gggaacctac gataagcgct 21240
   tcccggggtt catggccccc gataagctcg cctgtgccat tgtaaatacg gccggacgtg 21300
   agacgggggg agagcactgg ttggctttcg gttggaaccc acgttctaac acctgctacc 21360
   tttttgatcc ttttggattc tcggatgatc gtctcaaaca gatttaccag tttgaatatg 21420
   agggtctcct gcgccgcagc gctcttgcta ccaaggaccg ctgtattacg ctggaaaaat 21480
   ctacccagac cgtgcagggt ccccgttctg ccgcctgcgg acttttctgc tgcatgttcc 21540
   ttcacgcctt tgtgcactgg cctgaccgtc ccatggacgg aaaccccacc atgaaattgc 21600
   taactggagt gccaaacaac atgcttcatt ctcctaaagt ccagcccacc ctgtgtgaca 21660
   atcaaaaagc actctaccat tttcttaata cccattcgcc ttattttcgc tcccatcgta 21720
   cacacatcga aagggccact gcgttcgacc gtatggatgt tcaataatga ctcatgtaaa 21780
   caacgtgttc aataaacatc actttatttt tttacatgta tcaaggctct gcattactta 21840
   tttatttaca agtcgaatgg gttctgacga gaatcagaat gacccgcagg cagtgatacg 21900
   ttgcggaact gatacttggg ttgccacttg aattcgggaa tcaccaactt gggaaccggt 21960
   atatcgggca ggatgtcact ccacagcttt ctggtcagct gcaaagctcc aagcaggtca 22020
   ggagccgaaa tcttgaaatc acaattagga ccagtgcttt gagcgcgaga gttgcggtac 22080
   accggattgc agcactgaaa caccatcagc gacggatgtc tcacgcttgc cagcacggtg 22140
   ggatctgcaa tcatgcccac atccagatct tcagcattgg caatgctgaa cggggtcatc 22200
   ttgcaggtct gcctacccat ggcgggcacc caattaggct tgtggttgca atcgcagtgc 22260
   agggggatca gtatcatctt ggcctgatcc tgtctgattc ctggatacac ggctctcatg 22320
   aaagcatcat attgcttgaa agcctgctgg gctttactac cctcggtata aaacatcccg 22380
   caggacctgc tcgaaaactg gttagctgca cagccggcat cattcacaca gcagcgggcg 22440
   tcattgttag ctatttgcac cacacttctg ccccagcggt tttgggtgat tttggttcgc 22500
   tcgggattct cctttaaggc tcgttgtccg ttctcgctgg ccacatccat ctcgataatc 22560
   tgctccttct gaatcataat attgccatgc aggcacttca gcttgccctc ataatcattg 22620
   cagccatgag gccacaacgc acagcctgta cattcccaat tatggtgggc gatctgagaa 22680
   aaagaatgta tcattccctg cagaaatctt cccatcatcg tgctcagtgt cttgtgacta 22740
   gtgaaagtta actggatgcc tcggtgctcc tcgtttacgt actggtgaca gatgcgcttg 22800
   tattgttcgt gttgctcagg cattagttta aaagaggttc taagttcgtt atccagcctg 22860
   tacttctcca tcagcagaca catcacttcc atgcctttct cccaagcaga caccaggggc 22920
   aagctaatcg gattcttaac agtgcaggca gcagctcctt tagccagagg gtcatcttta 22980
   gcgatcttct caatgcttct tttgccatcc ttctcaacga tgcgcacggg cgggtagctg 23040
   aaacccactg ctacaagttg cgcctcttct ctttcttctt cgctgtcttg actgatgtct 23100
   tgcatgggga tatgtttggt cttccttggc ttctttttgg ggggtatcgg aggaggagga 23160
   ctgtcgctcc gttccggaga cagggaggat tgtgacgttt cgctcaccat taccaactga 23220
   ctgtcggtag aagaacctga ccccacacgg cgacaggtgt ttctcttcgg gggcagaggt 23280
   ggaggcgatt gcgaagggct gcggtccgac ctggaaggcg gatgactggc agaacccctt 23340
   ccgcgttcgg gggtgtgctc cctgtggcgg tcgcttaact gatttccttc gcggctggcc 23400
   attgtgttct cctaggcaga gaaacaacag acatggaaac tcagccattg ctgtcaacat 23460
   cgccacgagt gccatcacat ctcgtcctca gcgacgagga aaaggagcag agcttaagca 23520
   ttccaccgcc cagtcctgcc accacctcta ccctagaaga taaggaggtc gacgcatctc 23580
   atgacatgca gaataaaaaa gcgaaagagt ctgagacaga catcgagcaa gacccgggct 23640
   atgtgacacc ggtggaacac gaggaagagt tgaaacgctt tctagagaga gaggatgaaa 23700
   actgcccaaa acaacgagca gataactatc accaagatgc tggaaatagg gatcagaaca 23760
   ccgactacct catagggctt gacggggaag acgcgctcct taaacatcta gcaagacagt 23820
   cgctcatagt caaggatgca ttattggaca gaactgaagt gcccatcagt gtggaagagc 23880
   tcagccgcgc ctacgagctt aacctctttt cacctcgtac tccccccaaa cgtcagccaa 23940
   acggcacctg cgagccaaat cctcgcttaa acttttatcc agcttttgct gtgccagaag 24000
   tactggctac ctatcacatc ttttttaaaa atcaaaaaat tccagtctcc tgccgcgcta 24060
   atcgcacccg cgccgatgcc ctactcaatc tgggacctgg ttcacgctta cctgatatag 24120
   cttccttgga agaggttcca aagatcttcg agggtctggg caataatgag actcgggccg 24180
   caaatgctct gcaaaaggga gaaaatggca tggatgagca tcacagcgtt ctggtggaat 24240
   tggaaggcga taatgccaga ctcgcagtac tcaagcgaag catcgaggtc acacacttcg 24300
   catatcccgc tgtcaacctg ccccctaaag tcatgacggc ggtcatggac cagttactca 24360
   ttaagcgcgc aagtcccctt tcagaagaca tgcatgaccc agatgcctgt gatgagggta 24420
   aaccagtggt cagtgatgag cagctaaccc gatggctggg caccgactct cccagggatt 24480
   tggaagagcg tcgcaagctt atgatggccg tggtgctggt taccgtagaa ctagagtgtc 24540
   tccgacgttt ctttaccgat tcagaaacct tgcgcaaact cgaagagaat ctgcactaca 24600
   cttttagaca cggctttgtg cggcaggcat gcaagatatc taacgtggaa ctcaccaacc 24660
   tggtttccta catgggtatt ctgcatgaga atcgcctagg acaaagcgtg ctgcacagca 24720
   ccctgaaggg ggaagcccgc cgtgattaca tccgcgattg tgtctatctg tacctgtgcc 24780
   aaacgtggca aaccggcatg ggtgtatggc agcaatgttt agaagaacag aacttgaaag 24840
   agcttgacaa gctcttacag aaatctctta aggttctgtg gacagggttc gacgagcgca 24900
   ccgtcgcttc cgacctggca gacctcatct tcccagagcg tctcagggtt actttgcgaa 24960
   acggattgcc tgactttatg agccagagca tgcttaacaa ttttcgctct ttcatcctgg 25020
   aacgctccgg tatcctgccc gccacctgct gcgcactgcc ctccgacttt gtgcctctca 25080
   cctaccgcga gtgccccccg ccgctatgga gtcactgcta cctgttccgt ctggccaact 25140
   atctctccta ccactcggat gtgatcgagg atgtgagcgg agacggcttg ctggagtgtc 25200
   actgccgctg caatctgtgc acgccccacc ggtccctagc ttgcaacccc cagttgatga 25260
   gcgaaaccca gataataggc acctttgaat tgcaaggccc cagcagccaa ggcgatgggt 25320
   cttctcctgg gcaaagttta aaactgaccc cgggactgtg gacctccgcc tacttgcgca 25380
   agtttgctcc ggaagattac cacccctatg aaatcaagtt ctatgaggac caatcacagc 25440
   ctccaaaggc cgaactttcg gcctgcgtca tcacccaggg ggcaattctg gcccaattgc 25500
   aagccatcca aaaatcccgc caagaatttc tactgaaaaa gggtaagggg gtctaccttg 25560
   acccccagac cggcgaggaa ctcaacacaa ggttccctca ggatgtccca acgacgagaa 25620
   aacaagaagt tgaaggtgca gccgccgccc ccagaagata tggaggaaga ttgggacagt 25680
   caggcagagg aggcggagga ggacagtctg gaggacagtc tggaggaaga cagtttggag 25740
   gaggaaaacg aggaggcaga ggaggtggaa gaagtaaccg ccgacaaaca gttatcctcg 25800
   gctgcggaga caagcaacag cgctaccatc tccgctccga gtcgaggaac ccggcggcgt 25860
   cccagcagta gatgggacga gaccggacgc ttcccgaacc caaccagcgc ttccaagacc 25920
   ggtaagaagg atcggcaggg atacaagtcc tggcgggggc ataagaatgc catcatctcc 25980
   tgcttgcatg agtgcggggg caacatatcc ttcacgcggc gctacttgct attccaccat 26040
   ggggtgaact ttccgcgcaa tgttttgcat tactaccgtc acctccacag cccctactat 26100
   agccagcaaa tcccggcagt ctcgacagat aaagacagcg gcggcgacct ccaacagaaa 26160
   accagcagcg gcagttagaa aatacacaac aagtgcagca acaggaggat taaagattac 26220
   agccaacgag ccagcgcaaa cccgagagtt aagaaatcgg atctttccaa ccctgtatgc 26280
   catcttccag cagagtcggg gtcaagagca ggaactgaaa ataaaaaacc gatctctgcg 26340
   ttcgctcacc agaagttgtt tgtatcacaa gagcgaagat caacttcagc gcactctcga 26400
   ggacgccgag gctctcttca acaagtactg cgcgctgact cttaaagagt aggcagcgac 26460
   cgcgcttatt caaaaaaggc gggaattaca tcatcctcga catgagtaaa gaaattccca 26520
   cgccttacat gtggagttat caaccccaaa tgggattggc ggcaggcgcc tcccaggact 26580
   actccacccg catgaattgg ctcagcgccg ggccttctat gatttctcga gttaatgata 26640
   tacgcgccta ccgaaaccaa atacttttgg aacagtcagc tcttaccacc acgccccgcc 26700
   aacaccttaa tcccagaaat tggcccgccg ccctagtgta ccaggaaagt cccgctccca 26760
   ccactgtatt acttcctcga gacgcccagg ccgaagtcca aatgactaat gcaggtgcgc 26820
   agttagctgg cggctccacc ctatgtcgtc acaggcctcg gcataatata aaacgcctga 26880
   tgatcagagg ccgaggtatc cagctcaacg acgagtcggt gagctctccg cttggtctac 26940
   gaccagacgg aatctttcag attgccggct gcgggagatc ttccttcacc cctcgtcagg 27000
   ctgttctgac tttggaaagt tcgtcttcgc aaccccgctc gggcggaatc gggaccgttc 27060
   aatttgtgga ggagtttact ccctctgtct acttcaaccc cttctccgga tctcctgggc 27120
   attacccgga cgagttcata ccgaacttcg acgcgattag cgagtcagtg gacggctacg 27180
   attgatgtct ggtgacgcgg ctgagctatc tcggctgcga catctagacc actgccgccg 27240
   ctttcgctgc tttgcccggg aactcattga gttcatctac ttcgaactcc ccaaggatca 27300
   ccctcaaggt ccggcccacg gagtgcggat ttctatcgaa ggcaaaatag actctcgcct 27360
   gcaacgaatt ttctcccagc ggcccgtgct gatcgagcga gaccagggaa acaccacggt 27420
   ttccatctac tgcatttgta atcaccccgg attgcatgaa agcctttgct gtcttatgtg 27480
   tactgagttt aataaaaact gaattaagac tctcctacgg actgccgctt cttcaacccg 27540
   gattttacaa ccagaagaac gaaacttttc ctgtcgtcca ggactctgtt aacttcacct 27600
   ttcctactca caaactagaa gctcaacgac tacaccgctt ttccagaagc attttcccta 27660
   ctaatactac tttcaaaacc ggaggtgagc tccaaggtct tcctacagaa aacccttggg 27720
   tggaagcggg ccttgtagtg ctaggaattc ttgcgggtgg gcttgtgatt attctttgct 27780
   acctatacac accttgcttc actttcttag tggtgttgtg gtattggttt aaaaaatggg 27840
   gcccatacta gtcttgcttg ttttactttc gcttttggaa ccgggttctg ccaattacga 27900
   tccatgtcta gacttcgacc cagaaaactg cacacttact tttgcacccg acacaagccg 27960
   catctgtgga gttcttatta agtgcggatg ggaatgcagg tccgttgaaa ttacacacaa 28020
   taacaaaacc tggaacaata ccttatccac cacatgggag ccaggagttc ccgagtggta 28080
   cactgtctct gtccgaggtc ctgacggttc catccgcatt agtaacaaca ctttcatttt 28140
   ttctgaaatg tgcgatctgg ccatgttcat gagcaaacag tattctctat ggcctcctag 28200
   caaggacaac atcgtaacgt tctccattgc ttattgcttg tgcgcttgcc ttcttactgc 28260
   tttactgtgc gtatgcatac acctgcttgt aaccactcgc atcaaaaacg ccaataacaa 28320
   agaaaaaatg ccttaacctc tttctgttta cagacatggc ttctcttaca tctctcatat 28380
   ttgtcagcat tgtcactgcc gctcatggac aaacagtcgt ctctatccct ctaggacata 28440
   attacactct cataggaccc ccaatcactt cagaggtcat ctgggccaaa ctgggaagcg 28500
   ttgattactt tgatataatc tgcaacaaaa caaaaccaat aatagtaact tgcaacatac 28560
   aaaatcttac attgattaat gttagcaaag tttacagcgg ttactattat ggttatgaca 28620
   gatacagtag tcaatataga aattacttgg ttcgtgttac ccagttgaaa accacgaaaa 28680
   tgccaaatat ggcaaagatt cgatccgatg acaattctct agaaactttt acatctccca 28740
   ccacacccga cgaaaaaaac atcccagatt caatgattgc aattgttgca gcggtggcag 28800
   tggtgatggc actaataata atatgcatgc ttttatatgc ttgtcgctac aaaaagtttc 28860
   atcctaaaaa acaagatctc ctactaaggc ttaacattta atttcttttt atacagccat 28920
   ggtttccact accacattcc ttatgcttac tagtctcgca actctgactt ctgctcgctc 28980
   acacctcact gtaactatag gctcaaactg cacactaaaa ggacctcaag gtggtcatgt 29040
   cttttggtgg agaatatatg acaatggatg gtttacaaaa ccatgtgacc aacctggtag 29100
   atttttctgc aacggcagag acctaaccat tatcaacgtg acagcaaatg acaaaggctt 29160
   ctattatgga accgactata aaagtagttt agattataac attattgtac tgccatctac 29220
   cactccagca ccccgcacaa ctactttctc tagcagcagt gtcgctaaca atacaatttc 29280
   caatccaacc tttgccgcgc ttttaaaacg cactgtgaat aattctacaa cttcacatac 29340
   aacaatttcc acttcaacaa tcagcattat cgctgcagtg acaattggaa tatctattct 29400
   tgtttttacc ataacctact acgcctgctg ctatagaaaa gacaaacata aaggtgatcc 29460
   attacttaga tttgatattt aatttgttct tttttttttt atttacagta tggtgaacac 29520
   caatcatggt acctagaaat ttcttcttca ccatactcat ttgtgcattt aatgtttgcg 29580
   ctactttcac agcagtagcc acagcaaccc cagactgtat aggagcattt gcttcctatg 29640
   cactttttgc ttttgttact tgcatctgcg tatgtagcat agtctgcctg gttattaatt 29700
   ttttccaact tatagactgg atccttgtgc gaattgccta cctgcgccac catcccgaat 29760
   accgcaacca aaatatcgcg gcacttctta gactcatcta aaaccatgca ggctatacta 29820
   ccaatatttt tgcttctatt gcttccctac gctgtctcaa ccccagctgc ctatagtact 29880
   ccaccagaac accttagaaa atgcaaattc caacaaccgt ggtcatttct tgcttgctat 29940
   cgagaaaaat cagaaattcc cccaaattta ataatgattg ctggaataat taatataatc 30000
   tgttgcacca taatttcatt tttgatatac cccctatttg attttggctg gaatgctccc 30060
   aatgcacatg atcatccaca agacccagag gaacacattc ccctacaaaa catgcaacat 30120
   ccaatagcgc taatagatta cgaaagtgaa ccacaacccc cactactccc tgctattagt 30180
   tacttcaacc taaccggcgg agatgactga aacactcacc acctccaatt ccgccgagga 30240
   tctgctcgat atggacggcc gcgtctcaga acagcgactc gcccaactac gcatccgcca 30300
   gcagcaggaa cgcgcggcca aagagctcag agatgtcatc caaattcacc aatgcaaaaa 30360
   aggcatattc tgtttggtaa aacaagccaa gatatcctac gagatcaccg ctactgacca 30420
   tcgcctctct tacgaacttg gcccccaacg acaaaaattt acctgcatgg tgggaatcaa 30480
   ccccatagtt atcacccagc aaagtggaga tactaagggt tgcattcact gctcctgcga 30540
   ttccatcgag tgcacctaca ccctgctgaa gaccctatgc ggcctaagag acctgctacc 30600
   aatgaattaa aaaatgatta ataaaaaatc acttacttga aatcagcaat aaggtctctg 30660
   ttgaaatttt ctcccagcag cacctcactt ccctcttccc aactctggta ttctaaaccc 30720
   cgttcagcgg catactttct ccatacttta aaggggatgt caaattttag ctcctctcct 30780
   gtacccacaa tcttcatgtc tttcttccca gatgaccaag agagtccggc tcagtgactc 30840
   cttcaaccct gtctacccct atgaagatga aagcacctcc caacacccct ttataaaccc 30900
   agggtttatt tccccaaatg gcttcacaca aagcccaaac ggagttctta ctttaaaatg 30960
   tttaacccca ctaacaacca caggcggatc tctacagcta aaagtgggag ggggacttac 31020
   agtggatgac accaacggtt ttttgaaaga aaacataagt gccaccacac cactcgttaa 31080
   gactggtcac tctataggtt taccactagg agccggattg ggaacgaatg aaaataaact 31140
   ttgtatcaaa ttaggacaag gacttacatt caattcaaac aacatttgca ttgatgacaa 31200
   tattaacacc ttatggacag gagtcaaccc caccgaagcc aactgtcaaa tcatgaactc 31260
   cagtgaatct aatgattgca aattaattct aacactagtt aaaactggag cactagtcac 31320
   tgcatttgtt tatgttatag gagtatctaa caattttaat atgctaacta cacacagaaa 31380
   tataaatttt actgcagagc tgtttttcga ttctactggt aatttactaa ctagactctc 31440
   atccctcaaa actccactta atcataaatc aggacaaaac atggctactg gtgccattac 31500
   taatgctaaa ggtttcatgc ccagcacgac tgcctatcct ttcaatgata attctagaga 31560
   aaaagaaaac tacatttacg gaacttgtta ctacacagct agtgatcgca ctgcttttcc 31620
   cattgacata tctgtcatgc ttaaccgaag agcaataaat gacgagacat catattgtat 31680
   tcgtataact tggtcctgga acacaggaga tgccccagag gtgcaaacct ctgctacaac 31740
   cctagtcacc tccccattta ccttttacta catcagagaa gacgactgac aaataaagtt 31800
   taacttgttt atttgaaaat caattcacaa aatccgagta gttattttgc ctcccccttc 31860
   ccatttaaca gaatacacca atctctcccc acgcacagct ttaaacattt ggataccatt 31920
   agatatagac atggttttag attccacatt ccaaacagtt tcagagcgag ccaatctggg 31980
   gtcagtgata gataaaaatc catcgggata gtcttttaaa gcgctttcac agtccaactg 32040
   ctgcggatgg actccggagt ctggatcacg gtcatctgga agaagaacga tgggaatcat 32100
   aatccgaaaa cggtatcgga cgattgtgtc tcatcaaacc cacaagcagc cgctgtctgc 32160
   gtcgctccgt gcgactgctg tttatgggat cagggtccac agtgtcctga agcatgattt 32220
   taatagccct taacatcaac tttctggtgc gatgcgcgca gcaacgcatt ctgatttcac 32280
   tcaaatcttt gcagtaggta caacacatta ttacaatatt gtttaataaa ccataattaa 32340
   aagcgctcca gccaaaactc atatctgata taatcgcccc tgcatgacca tcataccaaa 32400
   gtttaatata aattaaatga cgttccctca aaaacacact acccacatac atgatctctt 32460
   ttggcatgtg catattaaca atctgtctgt accatggaca acgttggtta atcatgcaac 32520
   ccaatataac cttccggaac cacactgcca acaccgctcc cccagccatg cattgaagtg 32580
   aaccctgctg attacaatga caatgaagaa cccaattctc tcgaccgtga atcacttgag 32640
   aatgaaaaat atctatagtg gcacaacata gacataaatg catgcatctt ctcataattt 32700
   ttaactcctc aggatttaga aacatatccc agggaatagg aagctcttgc agaacagtaa 32760
   agctggcaga acaaggaaga ccacgaacac aacttacact atgcatagtc atagtatcac 32820
   aatctggcaa cagcgggtgg tcttcagtca tagaagctcg ggtttcattt tcctcacaac 32880
   gtggtaactg ggctctggtg taagggtgat gtctggcgca tgatgtcgag cgtgcgcgca 32940
   accttgtcat aatggagttg cttcctgaca ttctcgtatt ttgtatagca aaacgcggcc 33000
   ctggcagaac acactcttct tcgccttcta tcctgccgct tagcgtgttc cgtgtgatag 33060
   ttcaagtaca accacactct taagttggtc aaaagaatgc tggcttcagt tgtaatcaaa 33120
   actccatcgc atctaatcgt tctgaggaaa tcatccacgg tagcatatgc aaatcccaac 33180
   caagcaatgc aactggattg tgtttcaagc aggagaggag agggaagaga cggaagaacc 33240
   atgttaattt ttattccaaa cgatctcgca gtacttcaaa ttgtagatcg cgcagatggc 33300
   atctctcgcc cccactgtgt tggtgaaaaa gcacagctag atcaaaagaa atgcgatttt 33360
   caaggtgctc aacggtggct tccagcaaag cctccacgcg cacatccaag aacaaaagaa 33420
   taccaaaaga aggagcattt tctaactcct caatcatcat attacattcc tgcaccattc 33480
   ccagataatt ttcagctttc cagccttgaa ttattcgtgt cagttcttgt ggtaaatcca 33540
   atccacacat tacaaacagg tcccggaggg cgccctccac caccattctt aaacacaccc 33600
   tcataatgac aaaatatctt gctcctgtgt cacctgtagc gaattgagaa tggcaacatc 33660
   aattgacatg cccttggctc taagttcttc tttaagttct agttgtaaaa actctctcat 33720
   attatcacca aactgcttag ccagaagccc cccgggaaca agagcagggg acgctacagt 33780
   gcagtacaag cgcagacctc cccaattggc tccagcaaaa acaagattgg aataagcata 33840
   ttgggaaccg ccagtaatat catcgaagtt gctggaaata taatcaggca gagtttcttg 33900
   taaaaattga ataaaagaaa aatttgccaa aaaaacattc aaaacctctg ggatgcaaat 33960
   gcaataggtt accgcgctgc gctccaacat tgttagtttt gaattagtct gcaaaaataa 34020
   aaaaaaaaac aagcgtcata tcatagtagc ctgacgaaca gatggataaa tcagtctttc 34080
   catcacaaga caagccacag ggtctccagc tcgaccctcg taaaacctgt catcatgatt 34140
   aaacaacagc accgaaagtt cctcgcggtg accagcatga ataattcttg atgaagcata 34200
   caatccagac atgttagcat cagttaacga gaaaaaacag ccaacatagc ctttgggtat 34260
   aattatgctt aatcgtaagt atagcaaagc cacccctcgc ggatacaaag taaaaggcac 34320
   aggagaataa aaaatataat tatttctctg ctgctgttca ggcaacgtcg cccccggtcc 34380
   ctctaaatac acatacaaag cctcatcagc catggcttac cagacaaagt acagcgggca 34440
   cacaaagcac aagctctaaa gtgactctcc aacctctcca caatatatat atacacaagc  34500
   cctaaactga cgtaatggga gtaaagtgta aaaaatcccg ccaaacccaa cacacacccc  34560
   gaaactgcgt caccagggaa aagtacagtt tcacttccgc aatcccaaca ggcgtaactt  34620
   cctctttctc acggtacgtg atatcccact aacttgcaac gtcattttcc cacggtcgca  34680
   ccgccccttt tagccgttaa ccccacagcc aatcaccaca cgatccacac tttttaaaat  34740
   cacctcattt acatattggc accattccat ctataaggta tattattgat gatg        34794